# EUROPÄISCHE PATENTANMELDUNG

(11) **EP 1 316 255 A1**
(43) Veröffentlichungstag der Anmeldung: **04.06.2003**
(21) Anmeldenummer: 02090409.0
(22) Anmeldetag: 26.04.1999
(51) Int. Cl.: A01N 59/00

(54) **Verwendung von mikronisierten Mineralien als Pflanzenschutzmittel**

(62) Teilanmeldung aus: 99913546.0
(71) Anmelder: Lelas, Tihomir, 10040 Zagreb (HR)
(72) Erfinder: Lelas, Tihomir, 10040 Zagreb (HR)
(74) Vertreter: Omsels, Hermann-Josef

(57) **Zusammenfassung**

Die Erfindung beschreibt eine Verwendung von mikronisierten Mineralien, insbesondere Kalziten, als Pflanzenschutzmittel, wobei der Korngrößendurchmesser der mikronisierten Mineralien unter 0,5 µm liegt. Durch die erfindungsgemäße Verwendung besteht die Möglichkeit, die mikronisierten Mineralien direkt auf die Pflanzen zu stäuben und damit durch die mechanische Wirkung der mikronisierten Mineralien auf Insekten und Schädlinge eine Schädlings- und Insektenbekämpfung zu erreichen. Weiterhin können die mikronisierten Mineralien in Form einer wässrigen Lösung/Suspension auf Pflanzen gestäubt werden und somit einen Pilzbefall der Pflanzen mindern bzw. verhindern.

## Beschreibung

Die Erfindung beschreibt eine Vorrichtung zum Feinmahlen und Mikronisieren von mineralischen und/oder organischen Materialien mit einer kleinen Korngrößenverteilung unter Anwendung von Schlag- und Reibungsprozessen. Ferner werden neuartige Verwendungsmöglichkeiten dieser Vorrichtung für unterschiedlichste Bereiche des täglichen Lebens, bei dem es auf die Feinmahlung von Feststoffen, aber auch der Vermischung von festen und/oder flüssigen Stoffen ankommt, beschrieben.

Eine derartige Vorrichtung und ein entsprechendes Verfahren ist bereits aus der DE 197 55 921.2 bekannt. Die dortige Erfindung beschreibt ein Verfahren zur Verbesserung der Wirksamkeit von Wirkstoffen, die mindestens aus Mineralstoffen bestehen, indem diese Wirkstoffe einer tribomechanischen Aktivierung unterzogen werden, bei der die Oberfläche der behandelten Wirkstoffe vergrößert und deren Struktur zur Vergrößerung des chemischen Potentials destabilisiert wird. Die dazu beschriebene Vorrichtung weist mindestens drei Kranzreihen auf, die einander gegenläufig angetrieben werden, wobei auf jeder Kranzreihe schaufelartige Vorsprünge (Ventilatorschaufeln) befestigt sind. Insbesondere besteht die Vorrichtung zum Feinmahlen und Mikronisieren von Materialien aus einem Gehäuse mit zwei Rotoren, die jeweils mehrere, ineinandergreifende, einander gegenläufig mit gleicher Winkelgeschwindigkeit angetriebene Kränze aufweisen, die getrennt angesteuert werden, und wobei die Kränze hohl sind und in ihrem Innern eine Vielzahl von beidseitig an den Kranzwänden befestigten Ventilatorschaufeln tragen, die als Kollisionshindernis für die feinzumahlenden und mikronisierenden Materialien dienen, und wobei die Materialien aufgrund der im Inneren des Gehäuses herrschenden Zentrifugalkräfte von einem inneren Kranz in einen äußeren Kranz transportiert werden. Die Aktivierung der Mineralstoffe geschieht dadurch, daß in die Integrität der Kristallgitter eingegriffen wird, wodurch sich eine Art Beschädigung ergibt, die sich wiederum in Form einer Aktivierung, beispielsweise auch elektrischer Art, bemerkbar macht. Die DE 197 55 921.2 sieht dabei als vorteilhaft die Behandlung von Zeolithen an, die dort zum heilsamen Verzehr für Menschen beschrieben werden; auch Calcite für den Agrarbereich werden erwähnt. Die Nachteile der DE 197 55 921.2 sehen wie folgt aus:
■ die dortige Rotoren bestehen aus Kränzen, in welche die Ventilatorschaufeln beidseitig eingebaut sind, daß heißt sich wie die Rippen in einem Skelett von einem zentralen Mittelpunkt aus verzweigen;
■ die dortigen Ventilatorschaufeln sind aus mehreren Bestandteilen zusammengeschweißt;
■ die dortigen Ventilatorschaufeln werden auf ihrer Oberkante sehr schnell abgenutzt - bedingt durch den Abreibprozeß. Daraus resultierte zwangsläufig eine Reduzierung der Rotorengeschwindigkeit; denn je schneller sich die Rotoren drehen, desto größer ist die Abreibung. Auch bedingt die Form der Ventilatorschaufeln eine erhöhte Anlagerung des Ausgangsmaterials, was wiederum zu einer Gewichtszunahme der Schaufeln führte und letztlich zu einem erhöhten Energieverbrauch.
■ die durch die bekannte Vorrichtung zu erzielende Mikronisierung liegt bei 20 µ pro Teilchen, wobei nur ca. 78 % aller Teilchen diese Größenordnung erreichten;
■ In der DE 197 55 921.2 wird nicht erwähnt oder nahegelegt, warum die Verwendung von mikronisierten Zeolithen als pharmazeutisches Mittel vorteilhaft sein soll.

Ausgehend von diesem Stand der Technik ist es daher Aufgabe der vorliegenden Erfindung, eine verbesserte Vorrichtung zum Mikronisieren von Mineralstoffen und anderen Materialien zur schaffen, die einen höheren Wirkungsgrad der Mikronisierung aufweist, sowie neuartige Verwendungsmöglichkeiten der derart mikronisierten Materialien zur Verfügung zu stellen.

Gelöst wird diese Aufgabe durch die im Patentanspruch 1 aufgeführten Merkmale, nämlich insbesondere dadurch, daß die Rotoren aus Scheiben bestehen, auf denen die Ventilatorschaufeln einseitig befestigt angeordnet sind und wobei die Ventilatorschaufeln mit den Kränzen verbunden sind und in korrespondierende Kanäle auf der jeweils gegenüberliegenden Rotorenscheibe durchgreifen, die einen Materialdurchgang unter den Ventilatorschaufeln verhindern. Durch diese erfindungsgemäßen Maßnahmen wird eine wesentlich effizientere Mikronisierung erreicht unter gleichzeitiger Schonung der Vorrichtung selbst. Daß heißt, die üblicherweise mit der Feinmahlung einhergehende starke Abnutzung der Ventilatorschaufeln und damit der gesamten Kranzreihen, was zu erhöhtem Wartungsbedarf führt und sehr kostenintensiv ist, wird vermieden. Erreicht wird dies dadurch, daß durch die Kanäle die Abnutzung der Ventilatorschaufeln selbst minimiert wird, indem bewußt ein Verbleiben von mikronisiertem Material innerhalb der Kanäle in Kauf genommen wird, was wiederum zu einem erhöhten Widerstand an den Ventilatorschaufeln selbst führt und damit letztlich zu einem erhöhten Grad der Mikronisierung.

Die Vorteile der erfindungsgemäßen Vorrichtung lassen sich wie folgt darstellen:
■ Diese Feinmahlung wird durch kontrollierte Luftströmungen erzielt, die durch die Anwendung von neuartig konstruierten Ventilatorschaufeln hervorgerufen werden.
■ Durch die Auswahl der Einstellung und der Neigung der Ventilatorschaufeln wird eine turbulente Luftströmung erzeugt, welche die Effizienz des Mikronisierungsprozesses steigert. Die neu konstruierte Form dieser Ventilatorschaufeln verlängert deren Lebensdauer im Verhältnis zu den bekannten Ventilatorschaufeln bis zu 30 mal.
■ Durch den einfachen Austausch von abgenutzten Werkzeugen (Ventilatorschaufeln und Stiften) ist das Handling und die Instandhaltung vereinfacht. Bei herkömmlichen Einrichtungen war dies bislang nicht möglich. Es mußten immer ganze Kränze ausgetauscht werden.
■ Die Kanäle, in welchen die Ventilatorschaufeln durchgreifen, bilden ein geschlossenes Labyrinthsystem für die Materialverarbeitung, welches die Bewegung des Materials, das der Verarbeitung unterzogen ist, so kontrolliert, daß die Körnchen nicht ohne Schlag- und Reibungswirkung neben den Ventilatorschaufeln unverarbeitet vorbeifliegen, womit die Effizienz der Verarbeitung optimiert wird.
■ Die zu erzielende Mikronisierung liegt bei 98,72 % aller Teilchen unter 4,3 µ. Ein Anteil von 28,36 % aller Teilchen weist sogar einen Durchmesser von unter 0,5 µ auf. Mit keinem herkömmlichen Verfahren oder einer bekannten Vorrichtung konnten derartige Ergebnisse erzielt werden.

Das durch die erfindungsgemäße Vorrichtung mikronisierte Material weist vielerlei Vorteile für unterschiedlichste Verwendungsmöglichkeiten auf:

Die neuartige Vorrichtung ruft bei mineralischen Rohstoffkomponenten diverse chemische und chemisch-physikalische Veränderungen hervor. Die Effekte, welche durch dynamische Reibungsprozesse entstehen, verleihen diesen Mineralen neue Eigenschaften, die sich bei der Herstellung diverser Produkte technologisch und kommerziell nutzen lassen.

### 1. Verwendung von zermahlenen Mineralien als pharmazeutisch wirksames Mittel

Aus der Gruppe der Zeolithe hat sich das Mineral Heulandit/Klinoptylolith aufgrund seiner Eigenschaften als vorteilhaft herausgestellt, nämlich aufgrund seiner Fähigkeit zur Wasseraufnahme, seiner Selektivität und lonenaustauschkapazität, sowie seiner chemischen Zusammensetzung, welche zeigte, daß dieses Mineral für den menschlichen Genuß absolut unbedenklich ist. Die mineralogischen und chemischen Eigenschaften von Klinoptilolithen wurden untersucht und stellen sich wie folgt dar:

**Tabelle 1**

| **Komponente** | **Anteil [%]** | |
|---|---|---|
| | **von** | **bis** |
| SiO₂ | 61,96 | 67,17 |
| TiO₂ | 0,15 | 0,32 |
| Al₂O₃ | 12,46 | 15,12 |
| Fe₂O | 0,98 | 2,05 |
| MnO | spur | 0,05 |
| MgO | 1,30 | 1,96 |
| CaO | 3,03 | 4,35 |
| Na₂O | 0.70 | 1,11 |
| K₂O | 0,78 | 1,32 |
| H₂O bei 100°C | 4,05 | 4,74 |
| H₂O bei 1000°C | 7,56 | 9,56 |

Der Kalziumgehalt dieses Minerals deutet darauf hin, daß es sich dabei um ein Kalziumzeolith handelt, welches in einer tuffartigen Struktur gebildet ist, d. h. es handelt sich hier um eine Art des Minerals Klinoptilolith mit den Eigenschaften der Heulanditgruppe. Das gemessene Raumgewicht des untersuchten Minerals Klinoptiloithes bewegt sich in dem Bereich von 1,41 bis 1,43 g/cm³. Difraktometrische und thermo-gravimetrische Untersuchungen zeigten, daß in allen untersuchten Mustern ungefähr gleicher Zeolithgehalt besteht. Die Ergebnisse der Röntgenuntersuchungen zeigen die Anwesenheit von folgenden Mineralsorten: Heulandithe (Klinoptilolithe), sowie in weiterer Folge im wesentlichen Quarz, Sand, Plagioklass und in einer geringen Menge auch Biothythe. Die mikroskopische Untersuchung mittels Elektronenmikroskop zeigte, daß die Materialstruktur aus feinen Tuffkörnern besteht, welche eine homogene Isotropmasse, praktisch die Zeolithmaterie, darstellt. Der Gehalt dieser Materie bewegt sich grundsätzlich immer im Rahmen zwischen 70 % bis 85 %. Es ist in weiterer Folge die Anwesenheit von eckigen Quarzsegmenten festgestellt worden, sowie plagioklasse Sandkörner, welche in der Regel eine durchschnittliche Korngröße von 60 µ aufweisen. Die Untersuchung des Schmelzpunktes an 10 Mustern hat gezeigt, daß Klinoptilolithe bei einer Temperatur von 1260 - 1280° C schmelzen. Die festgestellte Härte laut Moss beträgt 3 - 3,5. Der Glühverlust beträgt:

**Tabelle 2**

| | Glühverlust [Gew.-%] | | | |
|---|---|---|---|---|
| H₂O | bei 100 °C | 3,34 | bis | 3,36 |
| H₂O | bei 300 °C | 5,42 | bis | 5,51 |
| H₂O | bei 500 °C | 2,60 | bis | 2,64 |
| H₂O | bei 1000 °C | 2,50 | bis | 2,51 |
| Insgesamt | | 13,86 | bis | 13,92 |

Ergebnisse der Absorbtionsuntersuchungen:

**Tabelle 3**

| Zeitraum | Wasser [Gew.-%] | | Benzol [Gew.-%] | |
|---|---|---|---|---|
| [h] | von | bis | von | bis |
| 1 | 4,61 | 4,62 | 7,64 | 7,65 |
| 2 | 8.74 | 8,75 | 9,33 | 9,33 |
| 3 | 10,75 | 10,77 | 9,45 | 9,47 |
| 4 | 11,10 | 11,11 | 9,51 | 9,54 |
| 17 | 13,44 | 13,45 | 9,54 | 9,54 |

Weitere Eigenschaften des untersuchten Klinoptilolithes:

| | | |
|---|---|---|
| 1. Druckfestigkeit | a)bei trockenem Zustand | max. 426 kg/cm³ |
| | | min. 361 kg/cm³ |
| | | mittel 391 kg/cm³ |
| | b) mit Wasser gesättigten Zustand | |
| | | max. 360 kg/cm³ |
| | | min. 253 kg/ cm³ |
| | | mittel 292 kg/cm³ |
| 2. Wasseraufnahme | | 23,35 Gew.-% |
| 3. Raumgewicht | | 1,37 g/cm³ |

Die Untersuchungsergebnisse der elektrischen Leitfähigkeit zeigten, daß die erfindungsgemäß behandelten Zeolithe bedeutend mehr Wasserstoffionen zu binden vermögen als die unbehandelten Zeolithe. Dies ist die unmittelbare Folge der Unterschiede in der Kristallstruktur der untersuchten Zeolithe, die durch die Feinmahlenung und Mikronisierung zustandegekommen sind. Die folgende Tabelle zeigt Beispiele für die Messungen der Leitfähigkeit und des pH der Suspension bei nicht behandelten und behandelten Zeolithen:

**Tabelle 4**

| Mischzeit der Suspensio^{a} | Konzentration an Zeolith (mg/ml H₂O) | pH^{b} (unaktiviertes Zeoith) | pH^{b} (erfindungsgemäß behandeltes Zeolith) | Leitfähigkeit^{c} (unaktiviertes Zeolith) µS/cm | Leitfähigkeit^{c} (erfindungsgemäß behandeltes Zeolith) µS/cm |
|---|---|---|---|---|---|
| 2 Std. | 20 | 7,35 | 7,48 | 157 | 147 |
| 2 Std. | 40 | 6,45 | 7,22 | 198 | 180 |
| 2 Std. | 60 | 6,81 | 7,35 | 307 | 280 |
| 20 Std. | 40 | 6,67 | 6,90 | 259 | 218 |
| 20 Std. | 80 | 6,96 | 7,40 | | |
| 48 Std. | 80 | 6,87 | 7,44 | | |

| | | | | | |
|---|---|---|---|---|---|
| ^{a} mit Magnetrührer, 70 - 100 RPM; | | | | | |
| ^{b} pH Messung unmittelbar nach der Zentrifugation (15 Minuten auf 7000 RPM), | | | | | |
| ^{c} Leitfähigkeitsmessung im Überstand unmittelbar nach der Zentrifugation (15 Minuten auf 7000 RPM), Leitfähigkeit des redestillierten Wassers beträgt 1.8 µS/cm | | | | | |

Die Ergebnis ist, daß der pH-Wert der unbehandelten zu den behandelten Zeolithe steigt, während die Leitfähigkeit abnimmt. Dies führt zu der überraschenden Erkenntnis, daß das behandelte Zeolith aufgrund der sinkenden Leitfähigkeit bestens dazu prädestiniert ist, als natürliches und physiologisch wirksames Puffermaterial eingesetzt zu werden, denn das Puffervermögen des behandelten Zeolithpulvers und die damit verbundene Beständigkeit des pH-Wertes führt dazu, daß das behandelte Material, z.B. auf seinem Weg durch den Verdauungstrakt, von der Magensäure nicht zur Gänze, sondern nur geringfügig gesättigt wird. Somit bleibt das behandelte Zeolith auch im Dünndarm aktiv, was letztlich ein wünscheswertes Ergebnis ist..

Die Interaktion von behandelten Zeolithmaterial bei dem Reaktionsmodell der Oxidation von L-Askorbaten/Askorbinsäure mit Nitrobenzol^{(a)} beträgt:

**Tabelle 5**

| Lösungsmittel | Nitrobenzol/M | L-Askorbinsäure/Askorbat_{TOT} /M | Erfindungsgemäß behandeltes Zeolith [mg/ml] | K_{obs}/s^{-1 (b)} | T/°C |
|---|---|---|---|---|---|
| Wasser, | 3,0 x 10⁻⁴ | 3,0 x 10⁻⁴ | - | 0,1599 (0,0112) | 10 |
| pH = 4,22 | 3,0 x 10⁻⁴ | 3,0 x 10⁻⁴ | 0,375 | | |
| Wasser, | 1,2 x 10⁻⁴ | 1,2 x 10⁻³ | - | 0,6148 (0,0309) | 25 |
| pH = 3,75 | 1,2 x 10⁻⁴ | 1,2 x 10⁻³ | 0,504 | 0,7537 (0,0112) | 25 |
| | 1,2 x 10⁻⁴ | 1,2 x 10⁻³ | 0,500 | 0,6673 (0,0144) | 25 |
| | | | (nichtaktiviertes | | |
| | | | Zeolith) | | |
| Wasser, | 1,2 x 10⁻⁴ | 1,8 x 10⁻³ | - | 0,9374 (0,0287) | 25 |
| pH = 6,63 | 1,2 x 10⁻⁴ | 1,8 x 10⁻³ | 0,502 | 0,7473 (0,0845) | 25 |
| | 1,2 x 10⁻⁴ | 1,8 x 10⁻³ | 0,500 | 0,7964 (0,0134) | 25 |
| | | | (nichtaktiviertes | | |
| | | | Zeolith) | | |
| Dioxan/Wasser | 1,2 x 10⁻⁴ | 1,8 x 10⁻³ | - | 0,2981 (0,0246) | 25 |
| 50/50 % v/v pH = 6,66 | 1,2 x 10⁻⁴ | 1,8 x 10⁻³ | 0,508 | 0,2247 (0,0293) | 25 |
| Dioxan/Wasser | 1,2 x 10⁻⁴ | 1,8 x 10⁻³ | - | 0,6675 (0,0111) | 25 |
| 50/50 % v/v | 1,2 x 10⁻⁴ | 1,8 x 10⁻³ | 0,505 | 0,6021 (0,0071) | 25 |
| 0,0784 M | | | | | |
| KH₂PO₄+NaOH pH = 6,50 | | | | | |
| Wasser 0,0784 M | 1,2 x 10⁻⁴ | 1,8 x 10⁻³ | | 1,8670 (0,0613) | 25 |
| KH₂PO₄+NaOH | 1,2 x 10⁻⁴ | 1,8 x 10⁻³ | 0,502 | 1,8732 (0,0133) | 25 |
| pH = 6,50 | | | | | |
| ^{a} Modellreaktion nach S. Ursic et al. *New J. Chem.* 1998, *221.* Mit erweiterten Untersuchungen der Interaktion von erfindungsgemäß behandeltem Zeolith. | | | | | |
| ^{b} Mittelwert von 3-5 Messungen | | | | | |

Überraschende Erkenntnisse und damit Vorteile lassen sich wie folgt darstellen:
■ Veränderung des pH-Wertes auf dem zellulärem Niveau, wie z. B. die Entsäuerung von Tumorzellen, welche grundsätzlich einen niedrigen pH-Wert aufweisen; dadurch werden die Antitumorwirkungen verursacht.
■ Selektive Proliferation von Aminosäuren, Peptiden, Oligonukleotiden usw.;
■ Veränderung des lonenaustausches auf den Zellmembranen;
■ Interaktion der Zellrezeptoren und somit Beeinflussung der Zellprozesse;
■ Stärkung der Abwehrkraft des Organismus infolge vorübergehender oder vollkommener Blockade der Carbo-Kationen und freien Radikale;
■ Transport von bioaktiven Molekülen (z. B. Zusammenwirkung mit Silibin (einem Pflanzenextrakt, wie auch Rosskastanienextrakt, Gin Seng und Petersilienextrakt) und/oder Askorbinsäure).
■ Aktivierung und Optimierung der Aktivität der Kalium/Natrium-Pumpe auf der Zellmembran; dies führt zu einer Gesundung und Optimierung des Stoffwechselvorgangs der Zellen.
■ Transport von Blutzucker vom Blut in die Zelle ohne Verwendung von Insulin.

Das erfindungsgemäß behandelte Zeolith wurde versuchsweise insgesamt 600 Personen oral und lokal verabreicht. Dabei wurden folgende medizinisch relevante Auswirkungen festgestellt, für die das erfindungsgemäß behandelte Material vorteilhaft ist:
a) Krebserkrankungen
   ■ Hautkrebs
   ■ Lymphdrüsenkrebs
   ■ Knochenkrebs
   ■ Brustkrebs
   ■ Eierstockkrebs
   ■ Gebärmutterhalskrebs
   ■ Prostatakrebs
   ■ Hodenkrebs
   ■ Dünn- und Dickdarmkrebs
   ■ Leber-, Milz-, Gallen-, Bauchspeichelkrebs
   ■ Lungen-, Bronchienkrebs
   ■ Magenkrebs
   ■ Gehirntumore und andere kanzerogene Erkrankungen
b) Herz- und Kreislauferkrankungen
   ■ Funktionsstabilisierung und Optimierung des Gefäßsystems
   ■ Verminderung der Venendurchlässigkeit
   ■ Elastazirierung von erweiterten Venen und Rückbildung von Hämorrhoiden
   ■ Verschwinden erweiterter Kapillaren
   ■ Stärkung der Herzmuskeln
   ■ Stabilisierung des Blut- und Herzdrucks
c) Reduzierung des Blutfettes
   ■ allmähliche Stabilisierung der Blutfettwerte (Cholesterol, Triglyzeride)
   ■ Globale Veränderung des Blutbildes in Richtung optimaler Werte
d) Stabilisierung des Stoffwechsels und des Verdauungssystems
   ■ Optimierung des Stuhlgangs
   ■ Sanierung von Gastritis, Magengeschwür und Zwölffingerdarmgeschwür
e) Heilung von rheumatischen Erkrankungen
   ■ Ischias
   ■ Diskopathie
   ■ Spondilose
   ■ rheumatische Athritis
   ■ Gicht usw.
f) Optimierung der Nierenfunktionen
   ■ Diurethische Wirkung
   ■ Beseitigung von Entzündungen (Nefritis)
   ■ Verbesserung der Filterfunktionen
g) Bekämpfung von Hautkrankheiten
   ■ Seborrhoe
   ■ Dermatitis
   ■ Herpes Simplex und Herpes Zoster
   ■ Psoiasis
h) Diabetes mellitus
   ■ Absenkung des Zuckerniveaus im Blut
   ■ Beseitigung von Beschwerden bei peripherer Zirkulation
i) Endokryne Drüsen
   ■ Optimierung des Hormonausstoßes
   ■ Korrekturen bei unvollständiger Funktionsfähigkeit der Drüsen
j) Heilung bei Wunden und Verbrennungen
   ■ Offene Beine (Ulzera Cruris)
   ■ Dekubitus
   ■ Brandwunden, schmerzstillend und desinfizierend
   ■ Schnittwunden, schmerzstillend und desinfizierend
k) Parodontose
   ■ Eliminierung von Mikroorganismen aus dem Zahnfleisch
   ■ Verschließung und Heilung von offenen Wunden im Mundbereich
l) Beseitigung neuropsychiatrischer Erkrankungen
   ■ Epileptische Erkrankungen
   ■ Schizophrenie
   ■ Alzheimerische Erkrankungen
   ■ Parkinsonische Erkrankungen
   ■ Neurosen und Depressionen
   ■ Allgemeine Stimmungsverbesserungen
m) Pilzerkrankungen
   ■ antimykotische Wirkung auf der Haut und Schleimhaut (ausgeschlossen Augen)
   ■ antimykotische Wirkung bei den inneren Organen
n) gynäkologische Erkrankungen
   ■ Papiloma Virus (HPW 31)
   ■ Chlamydia
   ■ Verbesserung des Reinheitsgrades des vaginalen Bereiches

Die Testpatienten mit den oben angeführten Indikationen haben das behandelte Material in Pulverform vermischt mit Wasser eingenommen. Die Dosierung betrug zwischen 1 g und 12 g täglich; die Frequenz der Einnahme betrug 3 bis 12 mal pro Tag. Die Dosierung betrug 0,5 g bis 1 g pro Einnahme mit einem Abstand von 1 - 6 Stunden zwischen der Verabreichung.

Verfolgung des Krankheitsablaufs bei Patienten, bei denen die Therapie mit dynamisch feingemahlenen und mikronisierten Zeolithen durchgeführt wurde:

### 1.1 Patienten mit kanzerogenen Erkrankungen

Im Rahmen einer Testreihe wurden in einer Zeitspanne von 5 Monaten 280 Patienten behandelt.

Zelebrale Tumore wurden bei 21 Patienten, die sich in der Terminalphase befanden, verfolgt. Die Patienten waren im schlechten Allgemeinzustand, unbeweglich und bekamen nur eine symptomatische Therapie, d. h. sie bekamen nur schmerzstillende, antidepressive Mittel. 3 - 4 Wochen nach Anfang der Einnahme des aufbereiteten Materials, haben sich deutliche Verbesserungen des allgemeinen Zustands gezeigt. Die Patienten hatten keine Symptome epileptischer Anfälle mehr; sie sind wieder beweglich geworden und einige waren fähig selbst zu lesen, sich zu waschen und normal zu kommunizieren. Die Verbesserung des Allgemeinzustands setzte sich fort, so daß nach 5 Monaten 14 Patienten keine Anzeichen mehr von Krebserkrankungen hatten.

Bei 40 Patienten mit primären Lungentumoren, die sich in der terminalen Phase befanden, wurde der Allgemeinzustand in der 3. und 4. Woche nach dem Einnahmebeginn deutlich verbessert. Die Schmerzen wurden deutlich gemindert, die Resperation und Beweglichkeit wurden beschwerdenfrei. Bei dieser Gruppe wurde nur ein einziger Patient aufgrund eines invasivkahektischen Anfalls verloren.

Ferner wurden 53 Patienten mit terminalen Krebserkrankungen des Verdauungstrakts mit ausgesprochen kahektischen Zustand klinisch verfolgt. Bei diesen Patienten trat die positive Wirkung etwas später ein (Woche 5 bis 7), wobei 4 Patienten in der Anfangsphase der Einnahme verstorben sind. Der Rest der Patienten hat sich gut erholt und nach 5 Monaten hatten nur wenige die Reste der Krankheitssymptome zu verzeichnen.

Bei mehr als 150 Patienten wurde die positive Wirkung des behandelten Materials in Zusammenwirkung mit klassischen Methoden (Chemotherapie, radiologische Therapie) deutlich festgestellt. Die negativen Auswirkungen der klassischen Methoden wurden deutlich verringert.

### 1.2 Lebererkrankungen, Hepatitiszirrhose

Bei 20 Patienten mit chronischen Hepatitis-Viren zeigte bereits eine täglich geringe Dose von 2 - 3 g des behandelten Materials, eine deutliche Verbesserung des Allgemeinzustands. Bereits 2 Wochen nach Anfang der Einnahme waren Müdigkeit und Blähungen nicht mehr vorhanden. In 30 Tagen nach Anfang der Therapie wurden Transaminasen im Blut (AST, HLT, GGT, AP) und Bilirubin vermindert. Die Marker von Hepatitis, DNA und RNA zeigten virusnegative Ergebnisse.

Bei dekompensierten Zirrhosen wurde bereits nach 7 Tagen eine Verbesserung des allgemeinen Zustandes und der Rückgang von Ascites beobachtet.

### 1.3 Diabetes mellitus

Bei 24 Patienten mit insulinunabhängigem Diabetes wurde eine deutliche Glukoseverminderung und Stabilisierung im Blut bereits nach wenigen Tagen der Einnahme festgestellt. Es wurden bei mehreren Patienten daneben anderen therapeutische Mittel abgesetzt, ohne daß dies zu nachteiligen Ergebnissen führte.

### 1.4 Neurodegenerative Erkrankungen

Sehr positive Auswirkungen wurden bei Multipler Sklerose (13 Patienten) in den Anfangsphasen der Erkrankung beobachtet, während bei Patienten in den Terminalphasen (9 Patienten) die weitere Entwicklung der Krankheit gestoppt wurde. Sie zeigten jedoch keine deutliche Besserung des Allgemeinzustandes.

Gute Ergebnisse wurden auch bei Neurodermitis (7 Patienten), muskulären Distrophien (4 Patienten), Parkinsonischen Krankheiten (5 Patienten), Alzheimerischen Krankheiten (7 Patienten), Arteriosklerose (6 Patienten) und anderen Krankheiten festgestellt.

### 1.5 Andere Krankheiten

Die Einnahme des dynamisch feingemahlenen und mikronisierten Zeoliths hat auch gute Eigenschaften bei der Symptomminderung bei chronischer Arthritis, insbesondere rheumatischer Arthritis, gezeigt. Es wurden auch Erfolge bei dermatologischen Erkrankungen wie z. B. Psoriasis, Lupus Erritematodesa, Wunden- und Verbrennungsheilung beobachtet.

### 1.6 wirksame Zusätze

Die Vermischung des dynamisch feingemahlenen und mikronisierten Zeoliths mit anderen Mitteln erbringt zusätzlich vorteilhafte Ergebnisse, die teilweise sogar zu einer Verstärkung der Wirkung führten. Nachfolgende Mischungen haben sich als vorteilhaft für bestimmte Indikationen erwiesen:
■ mit Gelee Royal (Bienenköniginfutter) für die Vitalitätsverbesserung
■ mit Knoblauchextrakt für die Absenkung des Blutdrucks
■ mit Baldrianextrakt für die Beruhigung
■ mit fein gemahlener Haselnuß für die Bekämpfung rheumatischer Erkrankungen
■ mit Roßkastanienextrakt für die Elastizierung erweiterter Venen
■ mit Petersilienextrakt für die Verbesserung von Diurethik
■ mit Fruchtzucker für die Verbesserung der Muskelresorption
■ mit Sylimarin/Sylibin für die Beseitigung von Leberbeschwerden
■ mit Proteinen für die antikahektische Wirkung

### 2. Verwendung von erfindungsgemäß gemahlenen und mikronisierten Zeolithen als Zusatz bei der Herstellung diverser Lebensmittel

Mit der Zugabe von dynamisch feingemahlenen und mikronisierten Zeolithen bei einigen Lebensmitteln sind überraschende Wirkungen erzielbar, welche durch klassische Verfahren oder additive Zugaben nicht zu erreichen sind.

Als Zugabe bei der Produktion von Bäckereiprodukten (beigemischt der Suspension von Mehl und Hefe), unterstützt die Aktivität der Hefe, beschleunigt ihren Wachstum und Entwicklung, wodurch die Teigfermentierung verkürzt und das Teigvolumen vergrößert wird. Dadurch erzeugt man Bäckereiprodukte mit besseren organoleptischen (Geschmack, Geruch und Aussehen) und reologischen Eigenschaften (Weichheit, Elastizität). Auch die Haltbarkeit wird verlängert. Solche Einwirkungen auf die Aktivität der Hefe waren auf eine andere Weise bislang nicht zu erzielen.

Durch die Zugabe bei der Herstellung von Frucht oder Gemüsesäften, wird ihre Stabilität erhöht (keine Satzentstehung bzw. keine Differenzierung von vermischten Komponenten). Ihre biologischen Werte werden durch die positive Einwirkung des Additivs auf den allgemeinen Gesundheitszustand des Organismusses angehoben.

Produkte wie Marmeladen, Konfitüren, Fruchtsirupe und einige Säfte beinhalten eine bedeutende Menge der Saccharose, welche diesen die Süße sowie die entsprechende reologischen und organoleptischen Eigenschaften verleiht, die aber auch eine konservierende Wirkung hat. Solche Erzeugnisse für die Ernährung von Diabetikern beinhalten diese Saccharose nicht, diese wird mit Sorbitol oder Hydrokoloiden und technischen Konservierungsmitteln ersetzt. Mit der Zugabe von behandelten Zeolithen wird die gefragte Konservierungswirkung erzielt (wegen Bekämpfung der Aktivität einiger Bakteriensorten). Die biologische Wertigkeit des Produktes wird angehoben (günstige Beeinflussung des behandelten Zeoliths auf die allgemeinen Gesundheitszustand). Somit wird das Addieren von chemischen Konservierungsmitteln nicht nötig.

Behandelte Zeolithe eignen sich auch als Hilfsmittel bei Prozessen der Entfärbung von Lebensmittel (bei der Herstellung von Ölen, Lecithin, Proteinhydrolisathen, Glukose und ähnliches), wo die Standardmittel wie z. B. Aktivkohle für die bessere Effizienz ersetzt werden.

Die Anwendung des behandelten Zeoliths als Hilfsmittel bei der Prozeßfiltrierung von Weinen und Fruchtsäften, sowie bei der Anhebung der Effizienz von Komponententrennung, bei Filtrationsprozessen, welche als Zwischenphasen bei diversen technologischen Prozessen für die Herstellung von Lebensmittelprodukten, Stand der Technik sind. Es ist jedoch nicht bekannt, daß Zeolithe für diese Zwecke in Verwendung sind.

### 3. Verwendung von erfindungsgemäß gemahlenen und mikronisierten Lebensmittelrohstoffen in der Lebensmittelindustrie

Durch die Zusammenstöße unter den Körnchen, welche in der erfindungsgemäßen Vorrichtung bearbeitet werden, und durch relative Bewegung der Oberfläche eines Körnchens über der Oberfläche eines anderen Körnchens, entstehen Schäden in Lebensmitteln, wie beispielsweise das Auseinanderfallen von großen Molekülen (Zellulose, Proteine, Stärke, Polysacharide). Dabei werden Veränderungen bei einigen Lebensmittelkomponenten beobachtet. Diese neuartige Verwendung des Feinmahlens von Lebensmittelrohstoffen führt zu eine Verkürzung des Produktionsprozesses; auch entstehen keine thermischen Schäden am Material und es besteht kein Bedarf für andere Additive (z.B. Säuren, Laugen, Enzyme).

Die Feinmahlung kann bei der Herstellung von Sirup aus Stärke verwendet werden, d. h. bei der Produktion von Konditoreiprodukten, sowie bei Proteinhydrolisaten, die als Zusätze bei der Herstellung von fertigen und halbfertigen Gerichten dienen; aber auch bei Prozessen der Klärung von Fruchtsäften, wobei diese Prozesse das Verfahren der Depektinisierung ersetzt.

Die äußere Schale bei den Stärkegranulaten, welche aus Amilopektin zusammengesetzt sind, wird durch die Feinmahlung stark geschädigt und größtenteils desintegriert. Die Moleküle des Amilopektins werden auf die kürzeren Ketten zerrissen, wobei einige Fraktionen, wie Glukose, Maltose und Dextrine, freigesetzt werden. Durch die erfindungsgemäßen Maßnahmen kann auf klassische Verfahren der Mehlvermahlung verzichtet werden, wodurch die Erzeugung von Mehl mit spezifisch physikalischen Eigenschaften ermöglicht wird. Dies bedeutet, daß die Säurehydrolyse von der Stärke zum Zwecke der Herstellung von Stärkesirup entfällt. Die Zugabe von Säuren ist nicht erforderlich, genauso wie die thermische Behandlung der Rohstoffe.

Durch das beschriebene Verfahren werden auch physikalische Eigenschaften der Rohstoffe verändert. Vor allem durch die Zerstörung und Schädigung von Komponenten des Ausgangsmaterials wird die durchschnittliche Korngröße vermindert. Es entstehen Körnchen mit gleichmäßiger Korngröße; die spezifische Oberfläche der Körnchen wird vergrößert. Die beschriebene Bearbeitung von Pulvern führt zu einer Erhöhung des Schüttgewichtes, sowie einer Verbesserung der Löslichkeit und Dispersionsfähigkeit. Dies ist wichtig für die Stabilisierung des Produktionsprozesses bei der Herstellung von dehydrierten Säften und Getränken, denn durch die beschriebene Bearbeitung erhalten die Pulver die Eigenschaften der Instant-Substanzen (Fruchtsaft, Kakao, Kaffee, Tee), was bedeutet, daß sie schnell und einfach in kalten Medien rekonstituiert werden.

Üblicherweise werden Instanteigenschaften von Lebensmittelprodukten durch das Verfahren der Aglomeration vorher befeuchteter Körnchen und ihrer Zwangstrocknung erreicht. Die Innovation bei diesen Verfahren liegt auch in der Tatsache, daß die Körnchen des Pulvers voneinander entfernt werden, wodurch ihre Wasserbindungskapazität und ihre Löslichkeit gesteigert werden.

Bei der Verarbeitung von halbflüssigen Materialien verbessert sich infolge der Zerkleinerung der unlöslichen Körnchen (z.B. Pektin, Zellulose) die Homogenität und Viskosität, welche und infolge besserer Vermischung der Komponenten. Dies bezieht sich speziell auf das Frucht- und Gemüsemus, Cremesuppen und ähnliches. Standardtechnisch wird für diese Zwecke das Homogenisierungsverfahren angewendet, wobei in Hochdruckhomogenisatoren unter Anwendung des Hochdrucks, die Zerkleinerung von unlöslichen Komponenten hervorgerufen wird. Das beschriebene Verfahren des dynamischen Feinmahlens und Mikronisierung ist effizienter, denn dadurch entstehen Körnchen mit kleineren Ausmaßen und besserer Verteilung, wodurch wiederum die Produkte mit höherer Stabilität, ohne Phasenseparierung und - segmentierung hergestellt werden.

Weitere Vorteile sehen wie folgt aus:
■ Es ist eine Erhöhung der Stabilität bei diversen Emulsionen (Soßen, Mayonnaisen, Dressings) zu beobachten. Falls z.B. Öl und Wasser vermischt werden und dann dem Verfahren unterzogen werden, entstehen Tropfen mit dispersen Phasen, mit kleinen Ausmaßen und Größengleichheit, und auch mit hohen Verteilungsgrad innerhalb von kontinuierlichen Phasen. Somit werden die Disposität und Stabilität der Emulsionen verbessert. Gleichzeitig wird die Dauer des Prozesses verkürzt und die Wirtschaftlichkeit verbessert. Denn üblicherweise wird ein solches Verfahren in Kolloidmühlen und/oder Hochdruckhomogenisatoren abgewickelt, wobei eine hohe Zugabe von Emulgations- und Stabilisationsmittel erforderlich ist.
■ Für die Herstellung von Suppen und Soßen wird durch die Mikronisierung die Homogenität verbessert (stabilere Emulsionen). Beim Einfrieren ist keine Phasentrennung zwischen den öligen und wäßrigen Phasen mehr zu beobachten. Das Grundproblem bei der Einfrierung dieser Produkte ist gerade die Phasentrennung (Fett/Wasser, fest/flüssig), welche standardtechnisch nur teilweise durch Homogenisieren in Hochdruckhomogenisatoren gelöst werden kann. Dieses herkömmliche Verfahren hat negative Auswirkungen auf den Geschmack des Produktes. Bei der neuartigen Verwendung ergibt sich keine Geschmacksveränderung. Es wird eine viel bessere Homogenität des Fertigproduktes erzielt.
■ Durch die erfindungsgemäße Behandlung von Molkepulver, Milchpulver oder dem Konzentrat von Molkeproteinen, entsteht ein teilweiser Zerriß und eine Veränderung der Konfiguration der Proteinkomponente. Es wurde gefunden, daß derart behandelte Rohstoffkomponenten die Gefriertemperaturen, sowie die Anhebung der Viskosität und Stabilität (Eiscreme, Kinderernährung) beeinflussen. Auf diese Weise hergestellte Produkte haben auch bessere organoleptische Eigenschaften (bessere Konsistenz, Glätte, cremiger Geschmack). Der Produktionsprozeß wird ökonomisch günstiger, denn durch das Verfahren kann auf die herkömmlichen Prozesse der Homogenisierung, Reifung, Mischung und Zerkleinerung verzichtet werden.
■ Vorteile sind bei dem Prozeß der Eismassenreifung zu beobachten, da der gesamte Vorgang aufgrund des besseren Vermischungsgrads bei einzelnen Komponenten, sowie die höhere Stabilität der Emulsion verkürzt wird.
■ Bei der Verarbeitung von Eiern (Melange oder Eidotter oder Eiweiß) werden die Eigenschaften der verarbeiteten Komponente für die Vorbereitung von Lebensmittelprodukten zur Vereinfachung verbessert. Das Eiweiß bildet überraschenderweise sehr stabil Schaum und das Eidotter hat einen besseren Emulgationseffekt. Aus der Melange kann viel mehr Cholesterol extrahiert werden, dies infolge der verursachten Reibung unter den Komponenten.
■ Bei der Herstellung von Produkten wie Ölen, Lecithin, Pigmenten, ätherischen Ölen und ähnlichem, ist die Extraktion mit Hilfe von organischen Lösungsmitteln eine der Grundoperationen. Mit der Einschaltung des beschriebenen Verfahrens erhöht sich die Extraktionseffiziens. Die Dauer des Prozesses wird verkürzt und die Qualität des Produktes künstlich beeinflußt. Die Ursache dafür ist die Oberflächenvergrößerung der Rohstoffe (weil die Rohstoffe feiner vermahlen werden). Dadurch wird der Kontakt zwischen Rohstoffen und den Lösungsmitteln, sowie die Schwächung der Verbindungen zwischen extrahierenden Komponenten und des Rohstoffes verursacht. Dies ermöglicht eine viel einfachere Lösung der Komponente und ermöglicht die Abwicklung des weiteren Prozesses bei niedrigeren Temperaturen.
■ Der herkömmliche Herstellungsprozeß der Schokolade ist durch die Abwicklung des Konchen-Prozesses sehr lang; er dauert zwischen 9 und 11 Stunden. Das Konchen ist ein Prozeß der Mischung und Reibung bei erhöhten Temperaturen, er dauert mehrere Stunden, dient für die Anhebung des Aromas, Erhöhung der Viskosität, sowie für die Verteilung der einzelnen Komponten der Schokoladenmasse. Durch die erfindungsgemäße Behandlung der Komponenten wie Zucker, Kakaopulver und Milchpulver, wird der Konchen-Prozeß wesentlich verkürzt, denn durch das Verfahren wird die Größe der Körnchen verfeinert. Die Körnchen werden gleichmäßiger verteilt; die Tropfen der Fettphase werden ebenfalls mikronisert und eine bessere Vermischung aller Komponenten wird erzielt. Die Zugabe der Emulgatoren wird hierdurch verringert oder kann vollständig ausbleiben.
■ Die Zermahlung von Kaffee- oder Kakaobohnen, geteilten Körnern, dehydrierten Getränken in der erfindungsgemäßen Vorrichtung weist überraschende Eigenschaften im Gegensatz zu herkömmlichen Mahlverfahren auf. Die Dauer des Mahlens wird erheblich verkürzt. Das Aroma, Aussehen, Farbe, Löslichkeit, Dispersionsfähigkeit des Produktes werden verbessert und die Dauer des technologischen Prozesses verkürzt.

### 4. Verwendung in der Kosmetikindustrie

Überraschende Wirkung zeigten die erfindungsgemäß behandelten Zeolithe auch bei der Verwendung für kosmetische Zwecke, sowie als Zusatz bei der Herstellung von kosmetischen Produkten; einerseits bei der direkten Anwendung eines Pulvers
■ durch Einreiben auf die trockene Haut zur Erhöhung des Feuchtigkeitsgehaltes
■ durch Auftragen auf die Haut für Peeling
■ durch Zurückbildung der Falten durch direktes Auftragen
■ durch Entfernung von Haarschuppen durch direkte Anwendung auf die Kopfhaut vor dem Haarewaschen

Aber auch als Zusatz für kosmetischen Produkten, wobei sich Beimengungen von 10 % bis 50 % als vorteilhaft erwiesen in:
■ Vaselincreme
■ kaltgepresstes Olivenöl
■ Gingerextrakt
■ Jojobaöl
■ Palmenöl
■ Kakaobutter
■ sowie bei anderen Cremen, Cremeextrakten und Shampoos

Ferner ist auch die Anwendung als Zusatz bei medizinisch-kosmetischen Produkten bevorzugt, nämlich
■ als Zugabe bei Zahnpasten (gegen Parodentose)
■ als Zugabe bei Cremen gegen Hauterkrankungen und Hautpilzen
■ als Zugabe bei Haar- und Körpershampoos und -gels gegen Schuppenflechte

### 5. Verwendung von zermahlenen Mineralien als Pflanzenschutzmittel

Mineralien, insbesondere Kalzite (aber auch Dolomite und anderen Kalziumund Magnesium-Karbonate), die mit der erfindungsgemäßen Verrichtung dynamisch feingemahlen und mikronisiert wurden zeigten überraschende Eigenschaften für die Beschleunigung und Optimierung des Pflanzenwachstums, die Verkürzung der Vegetationsperiode, die Widerstandsfähigkeit gegen Schädlinge, sowie eine Ertrags- und Qualitätssteigerung der Ernte. Verursacht wird dies durch eine ultrafeine Vermahlung des Minerals. Die Vermahlung (Granulometrie) des behandelten Materials ist dabei von folgenden Parametern abhängig:
■ Granulometrie des Augangsmaterials
■ Drehzahl (Geschwindigkeit) der Rotoren
■ Anzahl der Rotoren
■ Einstellung der Ventilatorschaufeln
■ Mögliche Wiederholung des Mahlvorganges

Diese Parameter bestimmen auch die Eigenschaften des verarbeiteten Materials, sowie auch die spezifische (Kontakt-) Oberfläche des Materials, Destabilisierung der Materialstruktur und Anhebung der chemischen Reaktionsfähigkeit. Die Zusammenstöße und die unplastische Reibung der Oberflächen des Materials führen zu einer erhöhten chemischen Reaktionsfähigkeit. Die Vorteile lassen sich wie folgt darstellen:
■ Austauschfähigkeit der Ionen (vorwiegend Kationen) wird deutlich angehoben (um etwa 300 % gegenüber dem herkömmlich zerkleinerten Material).
■ Da die Tracheen an Blättern und Nadeln der Pflanzen einen durchschnittlichen Durchmesser von 12µ haben, sind sie dazu geeignet, die mikronisierten Teilchen mit den Durchmessern von 0,0-10,0 µ in das Blattinnere aufzunehmen.
■ Bei der Spaltung von CaCO₃ und MgCO₃ zu Kalzium und Magnesium einerseits und CO₂ andererseits, wird die energetische Bilanz (Input) der Pflanze günstig beeinflußt, denn die Pflanze wird mit CO₂ so versorgt, daß die Extrahierung des CO₂ aus der Luft praktisch unnötig wird. Somit wird der energetische Output der Pflanze wesentlich vermindert und die Pflanze spart Energie. Die energetische Bilanz der Pflanze wird angehoben, denn die in Kalziten befindlichen Spurenelemente an Magnesium-, Eisen- etc. unterstützen die Neubildung von Chlorophyll, wodurch wiederum die Photosynthese effizienter wird.
■ Die Positivierung der energetischen Bilanz führt zur Beschleunigung des Pflanzenwachstums und des Reifungsprozesses.

Drei Wirkmechanismen des feingemahlener Minerals für den Schutz und die Bekämpfung von Schädlingen weisen Vorteile :

### i) Mechanische Wirkung gegen Insekten

Die Feinteilchen, die sich auf den Blattoberflächen ansammeln, drängen in die Atemwege, Tracheen und Augen der Insekten und verursachen Irritationen in der Weise, daß sie von den Flächen migrieren, welche mit dem behandelten Material besprüht sind. Bei mehrmaliger Wiederholung der Ausbringung des feingemahlenen Minerals (alle 7 - 14 Tagen) ist die Abwesenheit von Insekten permanent.

### ii) Wirkung gegen Pilzschädlinge

Diverse Pilzkrankheiten wie z.B. Pheronospora, Botritis, Oidium u.a. entstehen bei den Pflanzen hauptsächlich dann, wenn die Blätter oder Früchte (Weintrauben, Erdbeeren) noch einige N-Verbindungen, insbesondere Ammonium, enthalten. Da die lonenaustauschfähigkeit des behandelten Materials die Stickstoffverbindungen schnell bindet, sind die Pilze nicht in die Lage, sich mit diesen Stoffen zu ernähren. Ihnen fehlt jegliche Lebensgrundlage sich überhaupt auf den behandelten Pflanzen anzusiedeln.

### iii) Schnelle Vegetationsentwicklung

Da zwischen dem Vegetationsfortschritt der Pflanzen und der Ansiedlung von Schädlingen eine Korrelation besteht, wird durch die Beschleunigung des Pflanzenwachstums und -entwicklung diese Korrelation gestört, so daß die Schädlinge mit Verspätung versuchen, sich an den Pflanzen anzusiedeln, wobei die Pflanzen bereits so entwickelt sind, daß sie sich selber gegen diese erfolgreich wehren können.

Als Folge der mechanischen Aktivierung der Kornoberfläche, wird die Aufnahmefähigkeit von Ammoniumionen sowie lonenaustausch bis zu 300 %, im Vergleich zu der nicht-aktivierten Grundsubstanz, gesteigert. Diese Eigenschaft des behandelten Materials eignet sich für den Zweck der Bindung von unangenehmen Gerüchen. Das behandelte Material kann somit für die Bindung unangenehmer Gerüche organischen Ursprungs bei Schweine-, Kälber-, Puten- und Hühnerfarmen erfolgreich eingesetzt werden. Da die Geruchsbindung in sehr kurzem Zeitraum erfolgt, eignet sich das behandelte Material für die Aussprühung in Räumen, für die Tiermast oder -zucht. Es entsteht eine angenehmere Atmosphäre für Tier und Mensch.

Die Bindung der unangenehmen Gerüche hat zur Folge, daß der Stickstoffanteil bei organischen Abfällen nicht durch die Verdunstung oder Ausspülung verloren geht. Die Bindung des Stickstoffes erhöht nicht nur den Düngewert, es dient auch der Schonung der Umwelt, insbesondere des Bodens und Grundwassers, da die Stickstoffionen so gebunden sind, daß sie durch Regenwasser nicht in das Grundwasser gespült werden. Vorteile konnten bei Gülle, Stallmist und Hühnerjauche erzielt werden. Für den Zweck der Geruchsbindung bzw. Bindung der Stickstoffverbindungen (Ammonium, Nitrate, Nitrite, Kaptane) reicht eine Zugabe des behandelten Materials, die den Feststoffanteil der erwähnten Abfälle in der Masse entspricht. Für die Beschleunigung der Kompostierung und der Herstellung von mineralischorganischen Düngemitteln ist ein Feststoffverhältnis von 70 % : 30 %, zugunsten des behandelten Materials, bevorzugt.

### Ausführungsbeispiel 1

Um die Retentionskapazität von behandelten Kalziten zu bestimmen, wurde zunächst eine -Wassermenge von 10 Litern mit Ammonium (NH₄⁺), in einer Konzentration von 780 ppm, durch einen Papierfilter mit 200 g behandelten Kalziten, langsam durchgelassen. Die Konzentration der Ammoniumionen sank auf 35 ppm , also um 95,7 %.

### Ausführungsbeispiel 2

Um die Geruchsverminderung zu bestimmen, wurde ein Versuch mit 20 Litern Schweinegülle, mit der Zugabe von 200 g behandelten Kalziten, durchgeführt. Die behandelten Kalzite wurden der Gülle beigemengt und eingerührt. Dabei wurde eine sofortige Geruchsminderung festgestellt. Diese war so deutlich, daß die kaum eine Spur eines Gestankes wahrzunehmen war.

### Ausführungsbeispiel 3

Ein Großversuch wurde auf einer Schweinefarm mit 3000 Mastschweinen (im Alter von 4 Monaten) gestartet, wobei eine Flächenstreuung von den behandelten Kalziten, mit der Dosierung von 40 g/m² der Bodenfläche, vorgenommen. Auch bei dieser Prüfung wurde eine sofortige Atmosphärenveränderung festgestellt, wobei die Großbelastung um etwa 80 % - 85 % zurückging. Der Schweinestall wurde noch 90 Tage weiterbehandelt. Die Ausbringung der gleichen Menge erfolgte jeden dritten Tag. Man konnte dabei ein viel ruhigeres Verhalten der Tiere beobachten. Krankheiten blieben völlig aus.

### Ausführungsbeispiel 4

Ein Gemisch von behandelten Kalziten und Hühnermist, im Verhältnis (Gew.%) 50:50, wurde dem Kompostierungsprozeß ausgesetzt. Eine Kompostbeschleunigung von über 70 % konnte festgestellt werden. Bei dem Mischungsverhältnis von 30 % der behandelten Kalzite und 70 % des Hühnermists, wurde auch eine Verkürzung der Kompostierungsdauer von 70 % festgestellt. Die Anwendung dieser Gemische als Düngemittel bei diversen Pflanzen zeigte, daß die Wertigkeit dieser als sehr hoch bezeichnet werden konnte.

### Ausführungsbeispiel 5

Verwendung von behandelten Kalziten für die Ertragssteigerung und Pflanzenschutz im Weinbau. Ein Weingut in Jois am Neusiedler See im. Burgenland Österreich mit 14 Hektar Weinbergen wurde als Standortversuch für die Weinsorten Welsch-Riesling, Zweigelt, Pinot Grau, grüner Veltliner, Riesling Silvana, Alter: 15 bis 20 Jahre, Bodenbeschaffenheit: sandig bis sandiglehmig unter panonischem Klima mit sehr hohe Luftfeuchtigkeitausgewählt. Üblicherweise ist dort ein häufiger Befall von Pilzkrankheiten (Peronospora, Oidium, Botritis) zu beobachten. Als Vergleichsfläche diente ein benachbarter 500 Hektar Weinberg mit gleichen Weinsorten und ähnlichen Bodenverhältnissen. Die gesamte Bodenfläche wurde bei Versuchsbeginn mit 40 Kg Kalziten pro Hektar besprüht. Eine weitere Bodendüngung wurde während der gesamten Versuchszeit nicht mehr durchgeführt. Die Blattdüngungen erfolgten in Perioden vom 1. Mai bis 15 September jeden Jahres mit einer Menge von 5 Kg behandelten Kalziten pro Hektar. Die Korngröße der behandelten Kalzite betrug von 0,0 - 10,0 µ. Diese wurde mit je 800 Litern Wasser pro Hektar in Form feiner Beregnung ausgebracht.

Überraschenderweise konnte die Reifezeit im Vergleich zu den vorigen Jahren und im Vergleich mit benachbarten Weingärten deutlich verkürzt werden und dauerte ca. 3 Wochen weniger. Aus diesem Grund wurde die Qualität der Trauben und Most deutlich verbessert. Sie betrug je nach Sorte 3 - 4 Klosterneuburg-Grade (entsprechend 18 - 24 Öchsle-Grad mehr), so daß man bereits bei normalen Reifezeiten, Spätlesecharakter erzielte. Der Ertrag verbesserte sich um ca. 8 - 10 % je nach Weinsorte.

Ferner konnte überraschend eine Schädlingsbekämpfung festgestellt werden: Während der gesamten Testzeit wurden weder prophylaktische noch radikale herkömmliche Schädlingsbekämpfungen vorgenommen. Trotzdem gab es kein Auftreten von Pilzerkrankungen. Zum Vergleich mußten die benachbarten Weingärten, welche auf herkömmliche Art und Weise gedüngt wurden, trotz Prophylaxe 8 - 10 mal pro Saison die radikale Bekämpfung, inbesondere gegen Peronospora-Befall, durchführen.

Etwa ein Drittel der Gesamtfläche, verteilt durch alle Kulturen, besonders bei der Sorte Grüner Veltliner, wurde jahrelang von Chlorose (Chlorophyl-Mangel) heimgesucht. Etwa 5 % der Gesamtfläche wies auch die Symptome der Nekrose aus. Während der Gesamtversuchsperiode wurden keine Symptome beider Krankheiten festgestellt, d. h. die verursachenden Mängel wurden durch die behandelten Kalzite aufgehoben.

### 6. Verwendung in der Zigarettenindustrie

Das erfindungsgemäß behandelte Material zeigt überraschende Wirkungen im Hinblick auf die Minimierung gesundheitsschädlicher Inhaltsstoffe von Filterzigaretten.

Zigarettenrauch ist ein sichtbares, verdunstendes Produkt der Tabakverbrennung, der Additive und des Zigarettenpapiers, der gleich nach der Verbrennungszone entsteht und sich wie folgt zusammensetzt:
■ gasförmige Phase (CO, CO₂, O₂, H₂, N₂N-Oxyd, HCN, HCNS, NH₃, Aldehyde, Alkohole usw.),
■ starr-flüssige Phase, zusammengesetzt aus Wasser und in ihr ganz oder teilweise schmelzende Verbindungen (wie Nikotin und weitere Alkaloide), die während des Rauchens destillieren, verdunsten oder verbrennen.

Während der Rauch durch den Tabak zieht, kommt es zur Abkühlung und Kondensation, ein Teil des Rauches gelangt in die Mundhöhle. Wegen der Verringerung der möglichen negativen, gesundheitsschädlichen Folgen des Rauches wird bekanntermaßen als Mundstück der Zigarette ein Zigarettenfilter eingebaut.

Der klassische Filter ist der Zellulose-Acetat-Filter. Um die Effizienz des Filters zu vergrößern (Fähigkeit des Zurückhaltens, der Absorption starrflüssiger und gasförmiger Phasen des Rauches), werden Additive, wie es Aktivkohle, Zeolithe, Silikagel, Magnesiumsilikat und künstliches Hämoglobin sind, verwendet.

Bekannt sind Mehrfachfilter, Papierfilter, Kombinationen mehrerer Filter und Additiva. Die Effizienz zur Reduzierung schädlicher Komponenten eines einfachen (Zellulose-Acetat-) Filters beträgt 20 - 65 %; durch verschiedene neuartig entwickelte Kombinationen, Additiva und Formen der Filter wird die Effizienz auf ca. 75 % angehoben. Ein großer Marktanteil kommt heute schon den sogenannten Leicht-Zigaretten ("Lights" oder "Ultra") zu, die weniger Trockenrauch-Kondensat, Wasser, Nikotin, CO₂ und andere schädliche Komponenten enthalten oder verursachen. Beim Projektieren von Light-Zigaretten nutzt man die Ventilation am Filter (Verdünnung des Rauches), verlängertes Wickelpapier der Filter (skirt tipping) und eine Kombination verschiedener Zigarettenpapiere und verschiedener Tabake. Die Effizienz und die Selektierung des Filters (erhöhte Fähigkeit der Absorption bestimmter Komponenten), sind ein wichtiger Faktor beim Projektieren der Light-Zigaretten. Einer der wichtigen Faktoren bei der Konstruktion des Filters und auch der Zigarette, ist der Saugwiderstand (pressure drop), der wesentlich auf den Geschmack und die Leichtigkeit des Rauchens Einfluß hat. Viele Additive und Konstruktionslösungen vergrößern den Saugwiderstand und ungewollte Effekte beim Rauchen. Dies ist der einfache Grund, warum noch immer beim größten Teil die einfachen Zellulose-Acetat-Filter verwendet werden.

Das erfindungsgemäß behandelte Material erhöht überraschenderweise die Effizienz und Selektion der Filter, sowie ein Anhalten und eine Verbesserung des gewünschten Geschmackes der Zigarette; dabei wird der Saugwiderstand des Filters kaum verändert. Die behandelten Zeolithe weisen eine größere Absorptionstärke auf, was auf die Veränderungen im Kristallgitter zurückzuführen ist.

Vorteilhaft hat sich ein Filterzusatz bei Zellulose-Acetat Filtern als Pulver mit einer Korngöße von 0,2 bis 0,5 µ erwiesen.

Bei der Herstellung der Filter, kann man die erfindungsgemäß behandelten Zeolithe direkt auf die Zellulose-Acetat-Faser oder das Papier bzw. Crest aufbringen - wie einen Zusatz im Hohlraum eines Multifilters, kombiniert mit dem Dualfilter usw.

Filter, die Gegenstand eines kontrollierten Experiments waren, wurden durch Hinzufügen von erfindungsgemäß behandelten Zeolithen direkt auf die Zellulose-Acetat-Faser produziert, nachdem man Plastifikatoren (Triacetin) vor dem Formen der Filterstäbchen im Zylinder eingesetzt hat. Das Pulver wurde durch ein besonders ausgearbeitetes Dosimeter direkt auf die Faser aufgebracht. Dadurch war es möglich, fast einheitlich bis zu 70 mg des erfindungsgemäß behandelten Zeoliths pro Filterstäbchen hinzuzufügen. Diese Art der Zugabe des Pulvers beeinflußt die Arbeit der herkömmlichen Maschinen und das Formen der Filterstäbchen nicht wesentlich, da die Maschinen zur Herstellung der Filterstäbchen einen dafür vorgesehenen Raum im Arbeitsfluß für diese Applikation haben. Durch diese Art der Zugabe des Pulvers ändert sich die Technologie der Stäbchenproduktion nicht wesentlich. Das technologische Verfahren wird nur um den Preis der Additive erhöht.

Die durchschnittlichen Resultate der gemessenen physischen Filterparameter ohne Zusatz von erfindungsgemäß behandelten Zeolithen und mit Zusatz von erfindungsgemäß behandelten Zeolithen, werden nachfolgend dargestellt.

**Tabelle 6**

| Muster | Saugwiderstand [mm VS] | Filtermasse [mg] | Zugabe von erfindungsgemäß behandelten Zeolith | |
|---|---|---|---|---|
| | | | 12 cm Filter | 2 cm Filter |
| 0 | 390 | 721 | 0 | 0 |
| 1 | 392 | 750 | 3,0 | 0,50 |
| 2 | 395 | 765 | 5,0 | 0,83 |
| 3 | 395 | 766 | 7,6 | 1,27 |
| 4 | 410 | 773 | 10,0 | 1,66 |
| 5 | 440 | 782 | 68,0 | 11,30 |

Die Zugabe der Zeolithe hat sich nicht wesentlich auf den Saugwiderstand und auch nicht auf das Gewicht des 12 cm langen Filterstäbchens ausgewirkt.

Die erhaltenen Resultate sind Durchschnitte der Messungen von 500 Filterstäbchen von jedem Muster. Der Anteil des zugegebenen behandelten Zeoliths wurde aufgrund des Unterschiedes der erhaltenen Menge Asche beim Verbrennen der Filterstäbchen im Mufon-Ofen bei 525° C berechnet. Für die Verarbeitung der Zigarette wurde im wesentlichen herkömmliches Filterpapier ohne Ventilation und ein Tabakgemisch, welches bei der Herstellung von full flavour-Zigaretten des Typs American Blend verwendet wird. Die verarbeiteten Zigaretten wurden nach Gewicht und Saugwiderstand sortiert und auf der Rauchmaschine (Borgwaldt RM 20) nach dem ISO-Standard erprobt. Jedes Muster wurde 5 mal auf der Maschine erprobt. Die Menge an Trockenrauch-Kondensat (Total Particule Matter), Nikotin, CO₂, Wasser und Teer wurden bestimmt. Alle Analysen wurden nach dem geltenden ISO-Standard; die Menge an Nikotin im Trockenrauch-Kondensat durch Extraktion mit 2-Propanol und anschließender HPLC durchgeführt.

Die erhaltenen durchschnittlichen Resultate sind in der nachfolgenden Tabelle dargestellt, aus der ersichtlich ist, daß bei Erhöhung des zugegebenen erfindungsgemäß behandelten Zeoliths im Filter, sich der Anteil an Nikotin, Trockenrauch-Kondensat, Teer, Wasser und CO₂ im Hauptstrom des Zigarettenrauches verringert. Gesetzlich sind die Zigarettenhersteller verpflichtet, den Gehalt an Teer und Nikotin in der Zigarette anzugeben. Dies den Gehalt an Teer und Nikotin in der Zigarette anzugeben. Dies macht die Bedeutung der Wesensart des CO₂ nicht geringer, welches negative Einwirkungen auf den Respiratorzyklus des Organismus hat. Das erfindungsgemäß behandelte Zeolith beseitigt überraschenderweise offenbar selektiv die schädlichen Komponente im Hauptstrom des Zigarettenrauches. Unabhängig von der Menge des Zeoliths, das auf den Filter appliziert wurde, ändert sich der Widerstand der Zigarette nicht wesentlich, aber auch nicht die Verbrennungsgeschwindigkeit. Die Leichtigkeit und Annehmlichkeit des Rauchens wurde beibehalten. Die Degustationsprüfungen haben gezeigt, daß die Muster mit dem größten Anteil an dem erfindungsgemäß behandelten Zeolith (Muster 5 - 11,3 mg und Muster 4 -1,66 mg) einen verbesserten Rauchgeschmack haben und ein hervorgehobenes Aroma aufweisen, so daß sie allgemein angenehmer zu rauchen sind.

**Tabelle 7**

| | | **Muster** | | | | | |
|---|---|---|---|---|---|---|---|
| **Zusammensetzung** | **Einheit** | **0** | **1** | **2** | **3** | **4** | **5** |
| Erfindungsgemäß | Filter [mg] | 0 | 0,50 | 0,83 | 1,27 | 1,66 | 11,30 |
| behandeltes Zeolith | | | | | | | |
| Trockenrauch- | Zigarette [mg] | 14,8 | 14,2 | 13,5 | 13,2 | 12,9 | 10,8 |
| Kondensat | | | | | | | |
| Nikotin | Zigarette [mg] | 1,03 | 0,98 | 0,94 | 0,93 | 0,91 | 0,75 |
| CO₂ | Zigarette [mg] | 14,98 | 14,37 | 13,61 | 12,46 | 11,86 | 10,90 |
| Wasser | Zigarette [mg] | 1,18 | 1,05 | 1,09 | 0,95 | 0,73 | 0,68 |
| Teer | Zigarette [mg] | 12,6 | 12,2 | 11,5 | 11,3 | 11,3 | 9,4 |
| Tabakmasse in | [mg] | 764 | 759 | 770 | 787 | 752 | 764 |
| Zigarette | | | | | | | |
| Anzahl der Züge | Züge/Zigarette | 7,4 | 7,0 | 7,4 | 7,5 | 7,2 | 7,4 |
| Saugwiderstand | [mm VS] | 102 | 102 | 105 | 109 | 108 | 111 |
| der Zigarette | | | | | | | |

In der nachfolgenden Tabelle sind die gemessenen durchschnittlichen Werte der Filter nach dem Rauchen dargestellt. Die Menge an Trockenrauch-Kondensat wurde aus dem Differenz der Menge des Produktionsmaterials vor und nach dem Rauchen berechnet. Die Menge des Nikotins wurde durch Extraktion aus dem Filter in 2-Propanol und Wasser durch die Karl-Fisher Methode bestimmt. Die Resultate zeigen, daß die mit dem erfindungsgemäß behandelten Zeolith applizierten Filter, eine erhöhte Fähigkeit der Absorption von Trockenrauch-Kondensat, Nikotin, und Wasser haben, welches die vorangegangenen Messungen und Resultate unterstreicht.

**Tabelle 8**

| | | **Muster** | | | | | |
|---|---|---|---|---|---|---|---|
| **Zusammensetzung** | **Einheit** | **0** | **1** | **2** | **3** | **4** | **5** |
| Erfindungsgemäß | mg/Filter | 0,00 | 0,50 | 0,83 | 1,27 | 1,66 | 11,30 |
| behandeltes Zeolith | | | | | | | |
| Trockenrauch- | mg/Filter | 13,50 | 13,30 | 13,80 | 14,30 | 14,80 | 15,40 |
| Kondensat | | | | | | | |
| Nikotin | mg/Filter | 0,53 | 0,58 | 0,60 | 0,60 | 0,67 | 0,69 |
| Wasser | mg/Filter] | 1,01 | 1,02 | 1,03 | 1,12 | 1,05 | 1,12 |

Aus diesen Ergebnissen folgt, daß eine Beimengung des erfindungsgemäß behandelten Minerals von 0,5 mg (vgl. Muster 1) vorteilhaft ist, da der Anteil an Trockenrauch-Kondensat abnimmt. Auch geht aus dieser Tabelle hervor, daß ein verstärktes Zurückhalten von Nikotin im Filter zu beobachten ist.

Durch die Applikation des erfindungsgemäß behandelten Zeoliths auf Zellulose-Acetat-Faser von Zigarettenfiltern führt also zu einer verstärkten Retention schädlicher Komponenten des Hauptstromes des Zigarettenrauches, zufriedenstellenden physischen Eigenschaften, ohne aber negativen Einfluß auf die degustativen und Raucheigenschaften einer Zigarette zu bewirken. Der Zusatz von 0,50 mg des erfindungsgemäß behandelten Zeoliths pro Filter beseitigt 4 % Trockenrauch-Kondensat, CO₂ und Nikotin, sowie 11 % Wasser. Ein Zusatz von 11,30 mg vom erfindungsgemäß behandelten Zeoliths pro Filter beseitigt 27 % Trockenrauch-Kondensat, CO₂ und Nikotin, sowie 42 % Wasser aus dem Hauptstrom des Rauches.

Ferner ist vorteilhaft eine weitere Zugabe von Selen (bis 25 µg/Filter) zu dem erfindungsgemäß behandelten Zeolith, da dadurch die Absorption freier Radikale, die durch Verbrennungen entstehen, erhöht wird.

### 7. Verwendung in der Bauindustrie

Die erfindungsgemäße Vorrichtung weist Vorteile bei der Behandlung von Rohstoffkomponenten aller Art, insbesondere zur Verwendung in der Baubranche auf, nämlich bei
■ Sand aller Art, Wüstensand eingeschlossen
■ Ton aller Art
■ Kalksteine
■ Tuff und Tuffelsen
■ Asche und Lavaschlacke
■ Kies, usw.

Ferner sind mineralische Rohstoffkomponente sekundärer Herkunft ebenfalls bevorzugt:
■ Industrieschlacke und -asche
■ Asche und Pulver elektronischer Filter
■ Baumüll, -schutt
■ ausgesonderte Ziegel
■ Tonabbrand
■ Magnesitabraum, usw.

Im Vergleich mit nichtaktiviertem Material, zeigt fein gemahlenes und mikronisiertes Material mit der erfindungsgemäßen Vorrichtung bearbeitet, eine erhöhte freie Energie und eine erhöhte Reaktionsfähigkeit. Bevorzugt ist eine Korngröße des unbearbeiteten Materials zwischen 0 - 600 µ.

Für die Verbesserung der Bindemittel wird dem bearbeiteten Material eine geringe Menge hydratisierten oder ungelöschten Kalks beigegeben.

Durch Homogenisierung fein gemahlener und mikronisierter Rohstoffkomponenten (für den beschriebenen Versuch wurde Wüstensand aus Dubai ausgewählt), sowie hydratisierten Kalk und Wasser ist eine Mischung erhältlich, die durch Pressen oder Vibrieren in Schablonen geformt werden kann, die für diese Zwecke ausgesucht sind und dann durch das Verfahren der hydrothermalen Bearbeitung unter folgenden Bedingungen trocknet werden:
■ im Wasserdampf bei einer Temperatur bis 90° C
■ unter Bedingungen erhöhten Drucks mit Wasserdampf bei einer Temperatur bis 170° C

Dieses Verfahren der Herstellung von Baumaterial basiert auf der beschleunigten Reaktion zwischen SiO₂ und Al₂O₃ aus der Zusammensetzung des Baumaterials auf der einen Seite und Ca(OH)₂ aus Kalk auf der anderen.

Die Anteile der Zusammensetzung technologischer Parameter und die Eigenarten der fertigen Produkte, können mit folgenden Angaben beschrieben werden: Der Anteil an feingemahlenem und mikronisiertem Wüstensand beträgt 90,6 - 95,4 %; der Kalkanteil im Gemisch 4,6 - 9,4 %. Das Gemisch wird homogenisiert und Wasser wird im Verhältnis 1,0:0,08-0,12 (Gemisch:Wasser) dazugegeben. Das so vorbereitete Rohgemisch wird durch Pressen geformt; anschließend härtet es unter folgenden Bedingungen hydrothermal aus: Druck des Pressens: 40 - 90 MPa; Temperatur der Aushärtung: 90 und 170°C; Dauer der Aushärtung: 4,5 - 7,0 h.

### Ausführungsbeispiel

### a) Herstellung eines Ziegels bei einer Temperatur von 90°C:

Ein Ziegel der Größe 25,0 x 12,5 x 6,5 cm wird aus 3,779 kg erfindungsgemäß fein gemahlenen und mikronisierten Wüstensand, 0,200 kg hydratisierten Kalk und 0,400 kg Wasser hergestellt. Die Eigenschaften des späteren Ziegels resultieren aus dem Druck des Pressens, aus der Temperatur und der Dauer der Aushärtung:
■ Druck des Pressens 40 MPa
■ Temperatur der Aushärtung 90°C
■ Dauer der Aushärtung 4,5 h

Der Ziegel, der auf diese Weise hergestellt wurde, hat folgende Kennzeichen:
■ Festigkeit auf Druck
■ nach einem Tag 20,6 MPa
■ nach 28 Tagen 28,6 MPa
■ Volumenmasse 2040 kg/m³
■ Wasseraufnahme 15,80 %

### b) Herstellung eines Ziegels bei einer Temperatur von 170°C (Aushärtung im Autoklav):

Ein Ziegel der Größe 25,0 x 12,5 x 6,5 cm wird aus 3,779 kg erfindungsgemäß fein gemahlenen und mikronisierten Wüstensand, 0,200 kg hydratisierten Kalk und 0,400 kg Wasser hergestellt. Die Eigenschaften des späteren Ziegels resultieren aus dem Druck des Pressens, aus der Temperatur und der Dauer der Aushärtung:
■ Druck des Pressens 90 MPa
■ Temperatur der Aushärtung 170°C
■ Dauer der Aushärtung 7,0 h

Der Ziegel, der auf diese Weise hergestellt wurde, hat folgende Kennzeichen:
■ Festigkeit auf Druck
■ nach einem Tag 36,2 Mpa
■ nach 28 Tagen 38,4 Mpa
■ Volumenmasse 2040 kg/m³
■ Wasseraufnahme 14,50 %

Die gewünschten physikalisch-mechanischen Eigenschaften des Materials hängen unmittelbar vom beschriebenen Bearbeitungsverfahren, sowie auch von den ausgewählten technologischen Parametern, die beim Herstellungsverfahren angewendet werden, ab. Vergleichbar mit diesen Ergebnissen sind allenfalls Betonziegel, welche durch Zugabe von Zement (15-25 Gew.-%) hergestellt werden. Die erfindungsgemäße hergestellten Ziegel weisen keinerlei bauphysikalische Nachteile auf.

Ziegel, Dachziegel, Balken, Blöcke, Töpfe und andere keramische Produkte werden herkömmlicherweise aus solchen Tonarten hergestellt, in denen sich nicht verschiedene Bestandteile befinden, die dem Backverfahren der geformten Produkte schaden. So ruft zum Beispiel der erhöhte Anteil an Kalziumcarbonaten (CaCO₃) in der Beschaffenheit der Rohstoffkomponente Ton ein sogenanntes Aufblühen auf dem fertigen Produkt hervor, da sich die Backtemperatur im Temperaturbereich des Sinterprozesses erhöht ( in einem Bereich von 960 - 1200°C).

Überraschenderweise wurde gefunden, daß es bei der Behandlung von Ton in der erfindungsgemäßen Vorrichtung zu einem gravierenden "Schaden" innerhalb des Kristallgitters kommt, was zu einer mechanisch-bedingten Aktivierung der Oberfläche und der Struktur und zum Übergang eines Teiles der Kristallphase in eine amorphe Form und überhaupt zur Änderung des energetischen Zustandes des Rohstoffes führt. Es konnte nachgewiesen werden, daß dadurch Ton seine physikalischen und chemisch-physikalischen Eigenheiten ändert, was sich wiederum in einer Änderung des Temperaturbereichs des Backens ändert; auch verringert sich der Punkt des Sinterprozesses deutlich und das gesamte Verfahren der thermischen Bearbeitung wird verkürzt. Zum Nachweis hierfür wurde Ton mit einem erhöhten Anteil an CaCO₃ (3,8 %) eingesetzt, welcher normalerweise nicht für die Herstellung von Backsteinen benutzt wird. Der Rohstoff wurde in der erfindungsgemäßen Vorrichtung mikronisiert und feingemahlen. Das so vorbereitete Material wurde mit Wasser gemischt, bis es die optimale Feuchtigkeit erreicht hat und wurde anschließend durch das Pressverfahren geformt. Es wurde ein Backstein normaler Größe 6,5 x 12,5 x 25,0 cm angefertigt. Der Backstein wurde nach der Formung durch das Pressverfahren bei einer Temperatur bis 200°C 24 Stunden getrocknet und anschließend bei einer Temperatur von 560°C gebacken. Bei dieser Temperatur ist es gelungen, den Sinterprozeß durchzuführen. Diese Temperatur wurde 60 Minuten gehalten, nachdem der Abkühlungsprozeß Schrittweise begonnen hatte. Der gesamte Vorgang der Erhöhung der Temperatur, des Sinterprozesses, sowie der Abkühlung, dauerte 12 Stunden.

Als Kontrolle wurde ein Backstein normaler Größe 6,5 x 12,5 x 25,0 cm benutzt; als Rohstoffbasis wurde die gleiche Rohstoffkomponente Ton verwendet wie im zuvorigen Experiment, mit dem Unterschied, daß der Ton nicht mikronisiert wurde. Der Backstein mußte nach der Formung durch das Pressverfahren bei einer Temperatur von 240°C 48 Stunden getrocknet werden. Danach wurde er bei einer Temperatur bis 980° C gebacken, bei welcher der Temperaturbereich des Sinterprozesses erreicht wurde. Diese Temperatur mußte 120 Minuten gehalten werden, bis es möglich war, den Abkühlungsprozeß einzuleiten. Der ganze Vorgang der Erhöhung der Temperatur, des Sinterprozesses und der Abkühlung, dauerte 22 Stunden. Der Vorgang wurde 25 mal wiederholt, ohne daß es zu erheblichen Änderungen des Vorganges kam. Hinsichtlich der Qualität der hergestellten Muster wurden folgende Unterschiede festgestellt:

**Tabelle 9**

| Eigenschaft | Ziegel mit erfindungsgemäß bearbeitetem Ton | Ziegel mit normalem, unbearbeitetem Ton |
|---|---|---|
| Druckfestigkeit | 159 Kp/cm² | 83 Kp/cm² |
| Wasseraufnahme | 3,2 % | 5,4 % |
| Ausblühen | NEIN | JA |

Weitere vorteilhafte Maßnahmen sind in den übrigen Unteransprüchen enthalten. Die Erfindung ist in den anliegenden Zeichnungen dargestellt und wird nachfolgend näher beschrieben. Es zeigt:
- **Figur 1**: eine schematische Ansicht der erfindungsgemäßen Vorrichtung;
- **Figur 2**: einen vertikalen Schnitt durch die Vorrichtung;
- **Figur 3**: eine Rotorenscheibe;

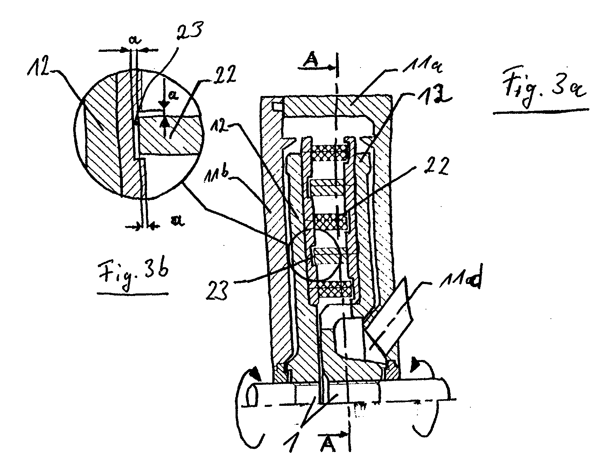

- **Figur 3 a**: einen vertikalen Schnitt durch einen Ausschnitt der zusammengesetzten Vorrichtung,
- **Figur 3b**: eine Ausschnittvergrößerung entlang eines Schnittes A-A gemäß der Fig. 3a;

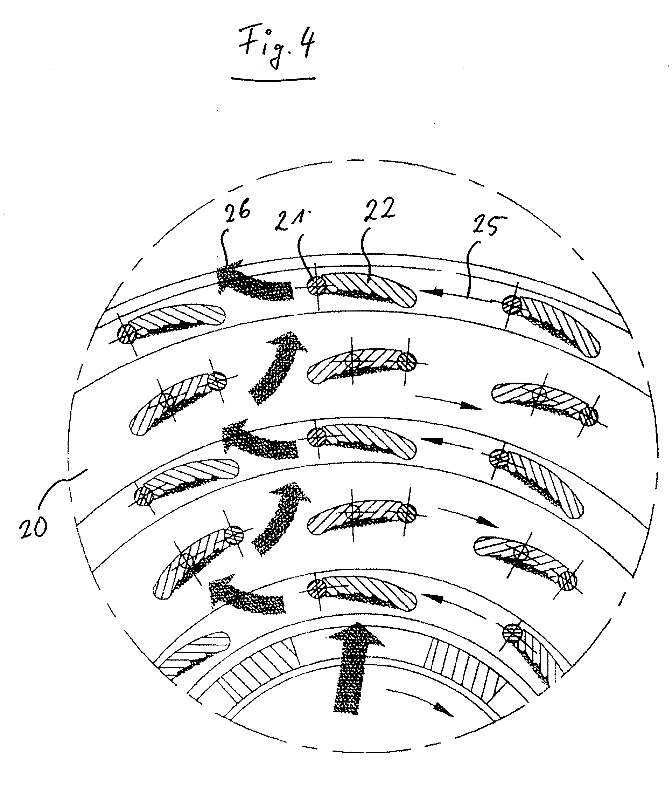

- **Figur 4**: die schematische Andeutung der Luftströme entlang der Ventilatorschaufeln;

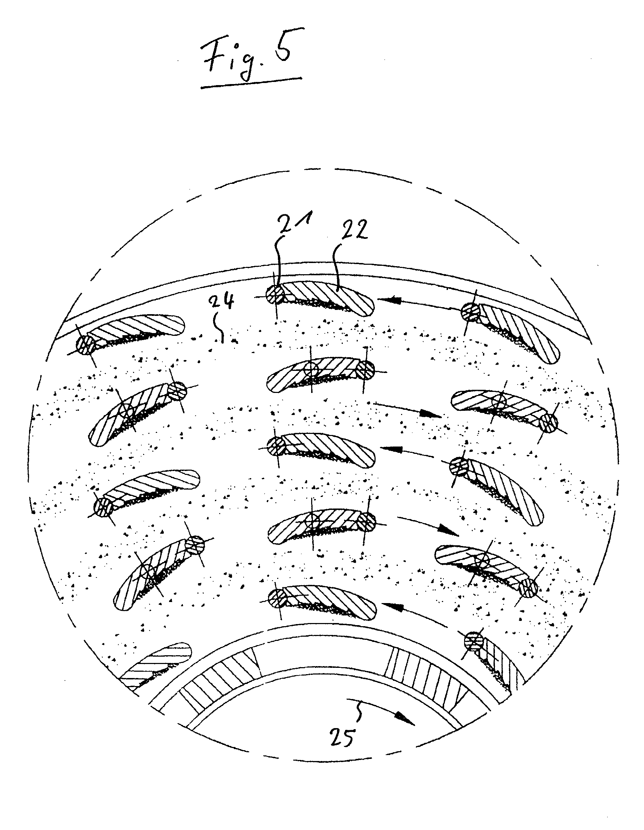

- **Figur 5**: die schematische Andeutung des zerkleinerten Materials entlang der Ventilatorschaufeln;

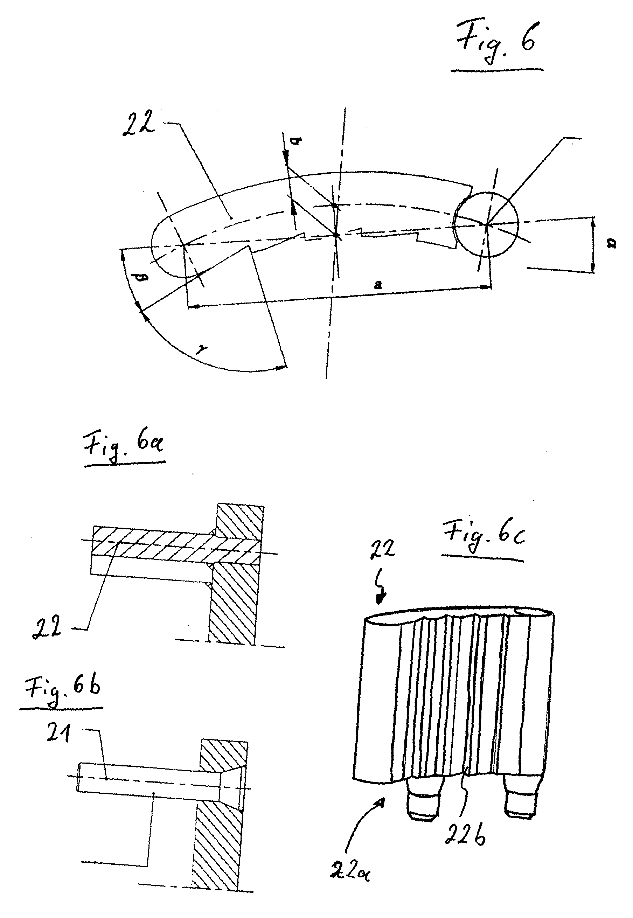

- **Figur 6**: eine Ventilatorschaufel mit Stift/Kanal im Detail und in mehreren Ansichten (**6a, 6b, 6c**);

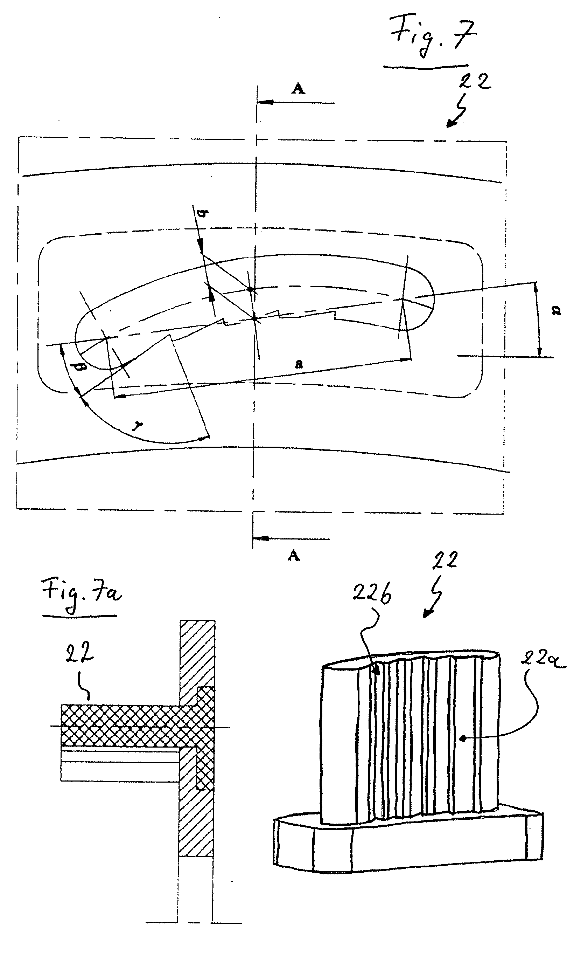

- **Figur 7**: schematische Darstellung einer Segmenten-Ventilatorschaufel;
- **Figur 7a**: Ausschnitt entlang eines Schnittes A-A gemäß Figur 7.

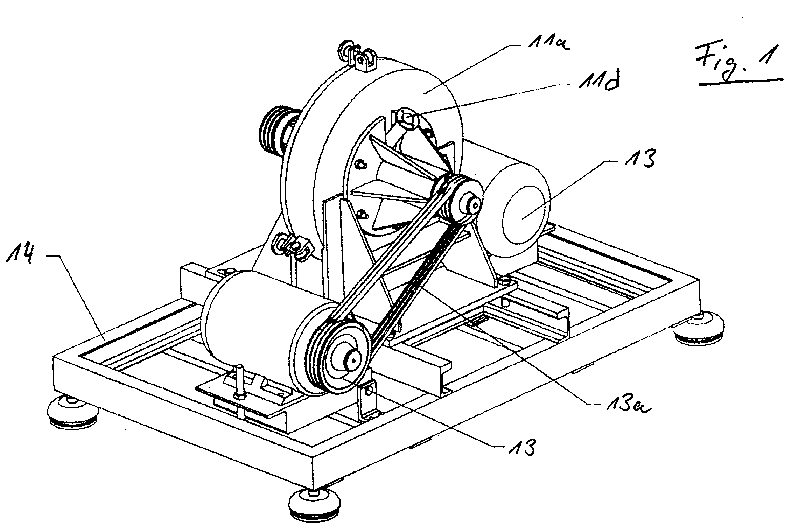

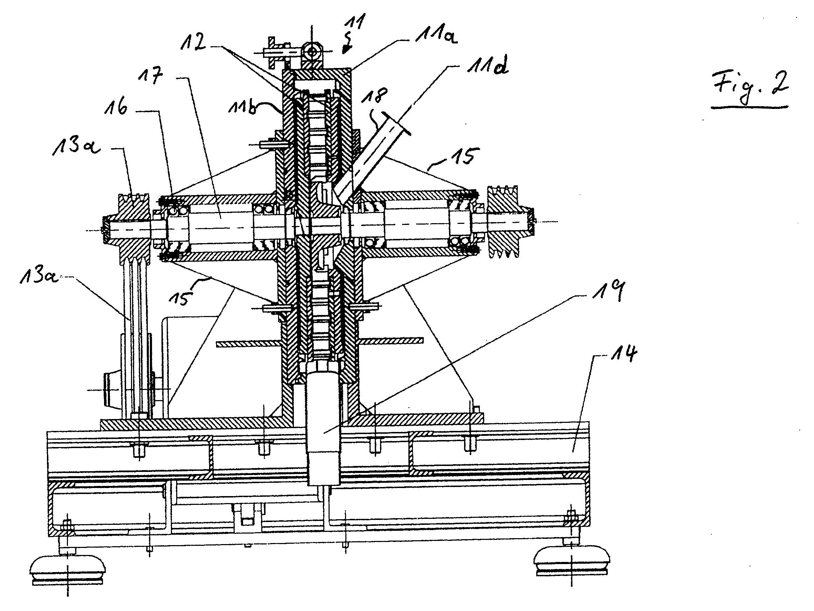

Die Figur 1 zeigt eine Vorrichtung 10 für die Feinmahlung und Mikronisierung, sowie das Homogenisieren von diversen festen und flüssigen Rohstoffkomponenten. Das Prinzip sieht derart aus, daß das Ausgangsmaterial durch die Mitte der Rotoren in den Verarbeitungsraum der Vorrichtung eingesaugt wird. Der Eintritt wird durch die Einwirkung von zentrifugalen Kräften in den Raum zwischen den Ventilatorschaufeln begünstigt und wird aufgrund der dort herrschenden Luftströme beschleunigt; so daß das Material mit dem bereits verarbeiteten Material kollidiert. Das Ausgangsmaterial wechselt die Bewegungsrichtung in sehr kurzen Intervallen; infolgedessen wird es zerkleinert und mikronisiert.

Die Vorrichtung 10 besteht aus einem aufklappbaren Gehäuse 11 mit einem Materialeinfuhrschacht 11d, in welchem sich zwei Rotorenscheiben 12 befinden, die gegeneinander gestellt sind und mittels entsprechender Motoren 13 über Riemen 13a gegenläufig betrieben werden, so daß sie sich mit gleicher Winkelgeschwindigkeit drehen. Das Gehäuse 11 und die Motoren 13 sind auf dem Fundament 14 befestigt und bilden eine unabhängige Einheit.

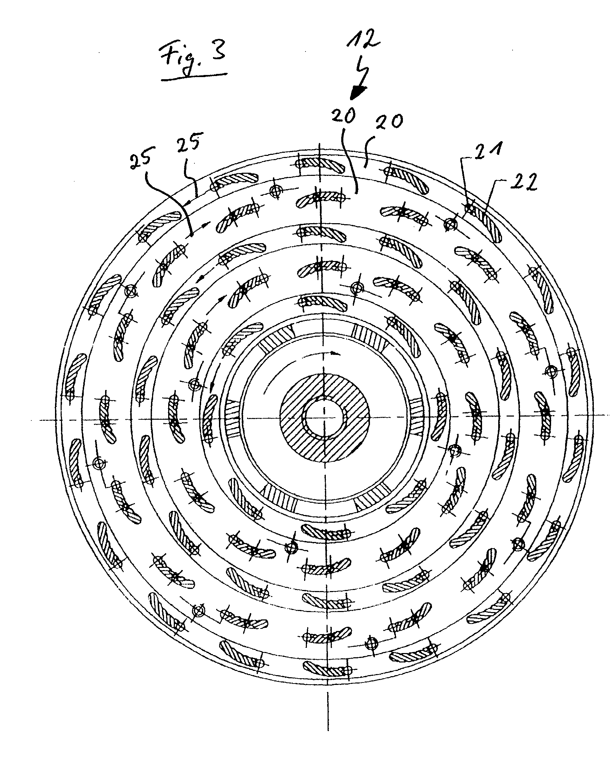

Figur 2 zeigt, daß das Gehäuse 11 aus zwei Teilen aufgebaut ist: eine Gehäuseseite 11a für die Materialeinfuhr und eine weitere Gehäuseseite 11b mit Einfuhrschacht 11c. Diese zwei Seiten 11a, 11b sind miteinander verschraubt. Auf beiden Seiten des Gehäuses 11 befinden sich die Träger 15, in welche die Lager 16 und Reckstangen 17 eingebaut sind. Auf der Seite 11a für die Materialeinfuhr befindet sich ein Rohr 18 für die geregelte Einfuhr des Materials; auf der unteren Seite befindet sich eine Öffnung 19 für den Ausstoß des fertigen Materials.

Figur 3, 4 und 5 zeigt, daß auf den Rotorenscheiben 12 mehrere konzentrische Kränze 20 mit den Stiften 21 und Ventilatorschaufeln 22 angeordnet sind, welche so konstruiert und ausgerichtet sind, daß sie berührungsfrei, während sie gegenläufig drehen, nebeneinander vorbeilaufen können - angedeutet durch die Drehrichtung 25. Minimal sind zwei Kränze erforderlich, die von den zwei Rotoren angetrieben werden. Die Aufgabe der Schlagstifte 21 und der Ventilatorschaufeln 22 ist die Erzeugung von turbulenten Luftströmungen für die Beschleunigung des verarbeiteten Materials, so daß Stöße und Reibung unter den Körnchen bei bestimmten Winkeln unter dynamischen Bedingungen hervorrufen werden. Die Kanäle 23 auf den Scheiben verhindern den Materialdurchgang unter den Ventilatorschaufeln 22, vgl. Figur 3 a und 3b.

Das Ausgangsgranulat (nicht dargestellt) wird, durch den Zentralteil 18 des Motorensystems durch Einsaugung eingebracht, durch die Luftströme 26 beschleunigt und so gesteuert, daß die Körnchen infolge mehrmaliger Bewegungsrichtung untereinander kollidieren und sich in sehr kurzen Zeitintervallen aneinander reiben. Dabei berühren sich die Arbeitswerkzeuge und andere Teile der Vorrichtung nicht oder nur geringfügig - aber es kommt auf keinen Fall zu Zerstörungen der Werkzeuge. Es entsteht eine Interaktion unter den Körnchen mit solchem Ausmaß, daß bei den Körnchen die innere Energie ausgetauscht wird, da die Zusammenstöße unplastisch sind (vgl. Fig. 4 und 5).

Die Effekte, die Folge der Zusammenstöße der Körnchen sowie Folge der relativen Bewegung der Oberfläche eines Körnchen über der Oberfläche von einem anderen Körnchen (mechanische Reibung) sind, werden verstärkt durch diejenigen Effekte, die infolge plötzlicher Richtungswechsel der Körnchenbewegung entstehen, so daß die Energie der Beschleunigung und relativer Bewegung der Körnchen in Energie der Deformierung, sowie in die Energie der molekularen Bewegung umgewandelt wird. Während der Zusammenstöße und der Reibung der Körnchen, welche dem Verkleinerungsprozeß in sehr kurzen Zeitintervallen (10⁻⁵ bis 10⁻⁶ s) unterzogen sind, entsteht eine bedeutende Veränderung ihrer Geometrie bzw. Form und Größe. Durch die relative Bewegung eines Körnchens über der Oberfläche eines anderen Körnchens, entstehen Schäden und Deformierungen der Körnchenoberfläche, sowie der Materialschichten, welche sich unmittelbar unter der Kornoberfläche befinden. Dadurch wird die Struktur des Kristallgitters auf der Oberfläche zerstört oder geschädigt, so daß teilweise die Kristallform in eine amorphe Phase umgewandelt wird, mit dem Ergebnis, daß die physikalischen und physikalisch-chemischen, sowie energetischen Eigenschaften des verarbeitenden Materials verändert werden. Bei solchen Verarbeitungen von Rohstoffkomponenten organischen Ursprungs zerreißen beispielsweise Zellulosefasern und große Molekülen werden mithin zu kleineren Molekülen, wobei diverse Veränderungen chemischer Zusammensetzung bei dem verarbeiteten Material hervorgerufen werden, sowie auch physikalische Veränderungen, die bedeutend für ihre weitere Materialverarbeitungen und/oder Aufbereitung, aber auch für die Wirksamkeit sind.

Neben der Veränderung der Eigenschaften des Materials, welches mit den beschriebenen Verfahren verarbeitet wird, wird dieses infolge von mechanischen Beanspruchungen fein gemahlen und mikronisiert und die Veränderung der granulometrischen Zusammensetzung des Materials ist von der Granulometrie der Ausgangskörner, sowie vom Niveau der Körnchenbeschleunigung, der geplanten Winkel der Zusammenstöße und gegenseitigen Reibung, sowie die geplanten Anzahl der Zusammenstöße abhängig. Folgende Parameter für die Feinmahlung und Mikronisierung mit der erfindungsgemäßen Vorrichtung stellen eine optimale Konfiguration dar, so daß diese Parameter bevorzugt sind:
■ Granulation des Ausgangskorns < 4,0 mm
■ Anzahl der Gesamtzahl der Kränze/Kranzreihen auf den Rotorenscheiben: 5
■ Durchmesser der Rotoren 500 mm
■ Drehzahl der Rotoren 3600/Min.
■ Kapazität der Vorrichtung 300Kg/Std.

Verglichen mit den Materialien, welche auf herkömmliche technische Weise (vgl. DE 197 55 921.2) feingemahlen und mikronisiert werden, zeigt das Material, welches durch die erfindungsgemäße Vorrichtung feingemahlen und mikronisiert wurde, eine erhöhte freie Energie und Reaktionsfähigkeit.

Die Innovation liegt mithin in der Konstruktion (Form, Verzahnung, Neigung) und Austauschbarkeit von Stiften und Ventilatorschaufeln auf die konstruierten Kanäle, die sich auf der Rotorenscheibe befinden, sowie in der Auswahl des Materials für die Ausarbeitung von Ventilatorschaufeln. Die Rotationsscheiben der Vorrichtung rotieren mit der gleichen Winkelgeschwindigkeit, bewegen sich jedoch gegenläufig. Das Ausgangsmaterial wird durch das Einsaugrohr 18 in den Zentralteil der rotierenden Scheibe eingeführt, aufgrund von Zentrifugalkräften werden die Körnchen der Rohstoffkomponente in Richtung des äußeren Randes des Gehäuses beschleunigt. Die Körnchen schlagen auf die Kränze 20 der Ventilatorschaufeln 22, welche sich in Gegenrichtung drehen. Sie wechseln die Bewegungsrichtung aufgrund der Richtungswechsel der Ventilatorschaufeln. Ferner schlagen und reiben sich die Körnchen untereinander, gehen nachfolgend in einen weiteren Kranz 20 mit Ventilatorschaufeln 22 über, wechseln wieder die Richtung der Bewegung im Einklang mit dem Richtungswechsel der rotierenden Scheibe, solange bis sie das Schaufelsystem verlassen. Am Ende der Bearbeitung in der erfindungsgemäßen Vorrichtung schlagen die Körnchen auf der Wand des Gehäuses auf und werden durch die Ausgangsöffnung 19 abtransportiert.

Figur 3a und 3b zeigt, daß die Kanäle 23 auf den Scheiben 12, in welche die Ventilatorschaufeln 22 durchgreifen, den Materialdurchgang unter den Ventilatorschaufeln 22 verhindern. Deren Form wird gemäß den Eigenschaften der jeweils zu verarbeitenden Rohstoffkomponenten (Materials) - also Granulation des Ausgangsmaterials, seine Feuchtigkeit, Härte, Ursprung, chemische Zusammensetzung und ähnliches - definiert. Wenn z. B. die Eingangsgranulation des Materials < 1 mm beträgt, bedeutet dies, daß der minimale Abstand zwischen den Ventilatorschaufeln und den Kanälen auf den Scheiben größer als 1 mm sein soll, um überhaupt das Durchgehen des Materials zu ermöglichen. Bei der Ausarbeitung und der Montage der Scheiben soll zufriedenstellende Parallelität verwirklicht werden, um den Ventilatorschaufeln das Durchgreifen in die Kanäle zu ermöglichen, denn die Durchmesser der Scheiben sind relativ groß (500 mm). Vorteile gegenüber der in der DE 197 55 921.1 beschriebenen Vorrichtung liegen darin, daß die Ventilatorschaufeln aufgrund ihrer Form, Neigung und Verzahnung bei dem Prozeß der Feinmahlung und Mikronisierung mit einer feinen Schicht des verarbeiteten Materials belegt werden und auf diese Weise von der Schlag- und Reibungswirkung des Eingangsmaterials geschützt werden. Die Abnutzung der Oberfläche der Ventilatorschaufeln wird somit minimiert und die Lebensdauer bedeutend verlängert. Ferner wird die energetische Ladung des Materials, welches behandelt wird, angehoben, wenn die Körnchen untereinander kollidieren und nicht mit den Teilen der Ventilatorschaufeln. Die technologischen Parameter, wie die Anzahl der Ventilatorschaufeln, ihre Neigung, die Form der Schaufelverzahnung, Anzahl der Ventilatorkränze, Winkelgeschwindigkeit der Scheiben, definieren die späteren Eigenschaften des verarbeiteten Materials. Mit der Kombination der angeführten Parameter wird es möglich, die Ergebnisse und Effekte zu programmieren.

In der Figur 3b sind weiter Scheibenkanäle (23) näher dargestellt. In den Rotorenscheiben (12) befinden sich an denjenigen Orten, die mit der Kranzreihe (20) des gegenüberliegenden Rotors (13) korrespondieren, Ausnehmungen (20a; siehe Fig. 3a, 3b), innerhalb derer die Ventilatorschaufeln (22) des gegenüberliegenden Kranzes (20) laufen. Die Kanäle (23) decken sich gegenseitig mit der Länge a, welche 2 - 5 mm beträgt. Sie bilden quasi ein Labyrinth, welches den Durchgang des Materials unter den Ventilatorschaufeln verhindert. Durch die Entstehung des Labyrinthes verstärkt sich der Widerstand für die Strömung unter den Ventilatorschaufeln. Somit wird erreicht, daß sich die Körnchen des Ausgangsmaterials durch die Hauptströmung zwischen den Ventilatorschaufeln bewegen. Die Form der Kanäle wird gemäß technisch-technologischer Eigenschaften der Rohstoffkomponenten des verarbeiteten Materials definiert (Granulation des Ausgangsmaterials, seine Feuchtigkeit, Härte, Ursprung, chemische Zusammensetzung und ähnliches). Wenn es die Scheibenkanäle nicht geben würde, würde sich das Ausgangsmaterial aufgrund der Zentrifugalkräfte so bewegen, daß es vom Zentrum bis zu der Peripherie der Scheiben an den Ventilatorschaufeln und der Scheibe vorbeigeht. Bei der Ausgangsgranulation des Materials von 0 - 1 mm muß die Entfernung zwischen den Ventilatorschaufeln und den Kanälen größer als 1 mm sein, um den Durchgang des Materials überhaupt zu ermöglichen, vorteilhaft hat sich eine Entfernung von 2 mm genauso wie für die Verdeckung a erwiesen.

Die Figuren 6, 6a, 6b und 6c zeigen die Geometrie der Ventilatorschaufeln 22 und der Stifte 21. Der Kranz 20, die Ventilatorschaufeln 22 und die Stifte 21 sind aus Hartstahl gebaut. Eine Alternative ist, den Stift aus Porzellan und die Ventilatorschaufeln aus Stahl herzustellen. Die Neigung der Ventilatorschaufeln 22 beträgt im Verhältnis zur Horizontalen α = 4 - 15° mit der Optimierung auf 8 - 10°. Die Anordnung und die Größe von der Verzahnung ist von der Anzahl der Ventilatorschaufeln abhängig (β = 30 - 120° und γ = 60 - 120°). Der Biegungsgrad der Ventilatorschaufeln ist mit dem Verhältnis der Längen a und b definiert, wobei b 10 % der Sehnenlänge darstellt. Die Anordnung der Ventilatorschaufeln ist fixiert. Sie sind in die Kranzform in entsprechende Ausnehmungen eingepreßt Die Form der Ventilatorschaufeln, profilierte Kollisionsoberfläche 22a, 22b und die Neigung sichern die Abfüllung der Ventilatorschaufeln mit dem Ausgangsmaterial und werden damit von der Abnutzungswirkung des Ausgangsmaterials geschützt, was auf die Verlängerung der Lebensdauer der Ventilatorschaufeln wirkt. Die Zähne 22b auf den Ventilatorschaufeln halten die erste Schicht des Ausgangsmaterials auf der Kollisionsoberfläche 22a, während die zweite Schicht langsam über die erste Schicht rutscht und diese zweite Schicht nimmt die Schläge des ankommenden Ausgangsmaterials an. Bevor die Ventilatorschaufeln 22 in die jeweilige Kranzreihe 20 der Rotorenscheibe 12 eingebracht wird, wird der Stift 21 in die Rotorenscheibe 12 eingepreßt. Die Symmetrieachse des Stiftes 21 befindet sich in der Symmetrieachse des Biegungsgrades der Ventilatorschaufeln 22, damit die Geometrie des Systems der Ventilatorschaufeln/Stifte optimal erreicht wird. Während der Materialverarbeitung schlagen die Materialkörnchen auf die Stirnseite der Ventilatorschaufeln und insbesondere auf und in die Stifte. Der Stift wird somit abgenutzt. Nach der Abnutzung wird der Stift durch Auspressen oder Bohren durch einen neuen ausgetauscht. Dies hat einen entscheidenden Vorteil: Bisherige Varianten der Ventilatorschaufeln ohne Stifte haben die Abnutzung der Ventilatorschaufeln selbst ergeben. Der Austausch der abgenutzten Ventilatorschaufeln ist im Vergleich mit dem Austausch der Stifte sehr kompliziert, aufwendig und teuer. Die Stifte können daher leicht ausgetauscht werden; die Ventilatorschaufeln bleiben unbeschädigt und müssen es nicht. De Austausch der Ventilatorschaufeln erfolgt erst bei allgemeiner Materialermüdung.

Das Problem der Vibrationen und der Festigkeit der Stifte ist dadurch gelöst, daß sich der Stift 21 mit der Fläche von 1/3 seines Stiftumfanges an eine korrespondierende Ausnehmung der Ventilatorschaufel 22 an diese Ventilatorschaufeln "anlehnt", und sich nicht geradflächig an die Ventilatorschaufeln anlehnt. Die Ventilatorschaufeln 22 werden durch Kaltpressen verformt oder auch durch Schmieden, wobei das Schmieden der besseren Aushärtung dient.

Eine andere Ausführungsform sieht erfindungsgemäß auch Segment-Ventilatorschaufeln (Figur 7, 7a) vor. Die Segment-Ventilatorschaufeln 22 werden aus Keramik oder Gußstahl hergestellt. Bei dieser Ausführungsform werden in die Rotorenscheiben 12 die Kanäle eingebaut, in welche die Segment-Ventilatorschaufeln mit unbestimmten Einlegungen gestellt werden. Die Genauigkeit der Einstellungen und die Festigkeit der Ventilatorschaufeln wird durch das Profil der Kanäle und durch die Reibung zwischen den Segment-Ventilatorschaufeln und die tragende Scheibe bestimmt. Die Neigung der Ventilatorschaufeln in Bezug auf die Horizontale beträgt α = 4 - 15° mit der Optimierung bei 8° - 10°. Die Einordnung der Zähne 22b und ihre Größe ist von der Länge der Ventilatorschaufeln 22 abhängig, Winkel β beträgt 30° - 120°, und Winkel γ beträgt 60° - 120°. Der Biegungsgrad der Ventilatorschaufeln ist mit dem Verhältnis der Längen a und b definiert, wobei b 10 % der Sehnenlänge darstellt. Die Form der Ventilatorschaufeln, der profilierten (verzahnten) Kollisionsoberfläche und der Neigung sichern, daß die Ventilatorschaufeln mit dem Ausgangsmaterial abgefüllt werden und damit von der Abnutzungswirkung des Ausgangsmaterials geschützt werden, was auf die Verlängerung der Lebensdauer der Ventilatorschaufeln wirkt. Die Zähne 22b auf den Ventilatorschaufeln halten wie in Figur 6 die erste Schicht des Ausgangsmaterials auf der Kollisionsoberfläche 22a, während die zweite Schicht langsam über die erste Schicht rutscht und diese zweite Schicht nimmt die Schläge des ankommenden Ausgangsmaterials an. Diese Ausführung hat im Vergleich zu obiger Ausführung (Fig. 6) den Vorteil, daß die Ventilatorschaufeln mit verschiedenen Neigungen (Winkel α) eingebaut werden können; somit ist der Austausch der Ventilatorschaufeln einfacher. Die Form der Ventilatorschaufeln hat keine Aussparung für die Stifte.

### Bezugszeichen

- 10: Vorrichtung
- 11: Gehäuse
- 11a: Gehäuseseite zur Materialeinfuhr
- 11b: Gehäuseseite mit Verschlußdeckel
- 11c: Verschlußdeckel
- 11d: Materialeinfuhrschacht
- 12: Rotorenscheiben
- 13: Motoren
- 13a: Riemen
- 14: Fundament / Gestell
- 15: Träger
- 16: Lager
- 17: Reckstangen
- 18: Einfuhrrohr
- 19: Auslaß
- 20: Kränze
- 20a: Ausnehmung
- 21: Stifte
- 22: Ventilatorschaufeln
- 22a: Kollisionsfläche
- 22b: Verzahnung
- 23: Kanäle
- 24: Zermahlenes Material
- 25: Drehrichtung
- 26: Luftströme

## Patentansprüche

1. Verwendung von mikronisierten Mineralien als Pflanzenschutzmittel, wobei der Korngrößendurchmesser der mikronisierten Mineralien unter 0,5 µm liegt.

2. Verwendung nach Anspruch 1, **dadurch gekennzeichnet, dass** die mikronisierten Mineralien auf die Pflanzen gestäubt werden.

3. Verwendung nach Anspruch 1, **dadurch gekennzeichnet, dass** die mikronisierten Mineralien in Form einer wässrigen Lösung/Suspension auf die Pflanzen aufgebracht werden.

4. Verwendung nach Anspruch 3, **dadurch gekennzeichnet, dass** die Konzentration der mikronisierten Mineralien in der wässrigen Lösung/Suspension bevorzugt bei 6.25mg/ml liegt.

5. Verwendung nach mindestens einem der Ansprüche 1 bis 4, **dadurch gekennzeichnet, dass** das Mineral ein Kalzit ist.
